# EUROPÄISCHE PATENTSCHRIFT

(11) **EP 0 865 609 B1**
(45) Veröffentlichungstag und Bekanntmachung des Hinweises auf die Patenterteilung: **19.03.2003**
(21) Anmeldenummer: 96942362.3
(22) Anmeldetag: 05.12.1996
(51) Int. Cl.: G01N 33/574

(54) **VERFAHREN ZUR DIAGNOSE UND THERAPIE VON PLATTENEPITHELKARZINOMEN**
METHOD OF DIAGNOSING AND TREATING EPITHELIOMA
PROCEDE POUR DIAGNOSTIQUER ET TRAITER LES EPITHELIOMES

(30) Priorität: 06.12.1995 DE 19545472; 17.04.1996 DE 19615074
(43) Veröffentlichungstag der Anmeldung: 23.09.1998
(73) Patentinhaber: Boehringer Ingelheim International GmbH, 55218 Ingelheim am Rhein (DE); Forschungszentrum Karlsruhe GmbH, 76021 Karlsruhe (DE)
(72) Erfinder: HEIDER, Karl-Heinz, A-1120 Wien (AT); ADOLF, Günther, A-1070 Wien (AT); OSTERMANN, Elinborg, A-1140 Wien (AT); PATZELT, Erik, A-3002 Purkersdorf (AT); SPROLL, Marlies, A-1120 Wien (AT)
(74) Vertreter: Laudien, Dieter, Dr.
(86) Internationale Anmeldenummer: EP9605448
(87) Internationale Veröffentlichungsnummer: WO97021104

(56) Entgegenhaltungen:
- WO-A-95/00658
- WO-A-95/00851
- WO-A-95/33771
- DE-A- 4 326 573
- AMERICAN JOURNAL OF PATHOLOGY, Bd. 146, Nr. 5, Mai 1995, Seiten 1102-1112, XP000197160 BROOKS ET AL.: "The expression of variant CD44 in nasopharyngeal carcinoma ...." in der Anmeldung erwähnt
- CANCER IMMUNOL IMMUNOTHER, Bd. 43, 1996, Seiten 245-253, XP000197161 HEIDER ET AL.: "Characterization of a high-affinity monoclonal antibody specific for CD44v6 ...." in der Anmeldung erwähnt

## Beschreibung

Die Erfindung betrifft Verfahren zur Diagnose und Therapie von Plattenepithelkarzinomen, die auf der Expression des variablen Exons v6 des CD44-Gens beruhen, Mittel für solche Verfahren sowie deren Verwendung.

Es wurde kürzlich gezeigt, daß die Expression von Varianten des Oberflächen-Glykoproteins CD44 notwendig und hinreichend ist, um sogenanntes spontanes metastatisches Verhalten sowohl in einer nicht-metastasierenden Pankreas-Adenokarzinom-Zellinie der Ratte als auch in einer nicht-metastasierenden Fibrosarkom-Zellinie der Ratte auszulösen (Günthert *et al*., 1991). Während die kleinste CD44-Isoform, die Standardform CD44s, in einer Reihe verschiedener Gewebe, darunter Epithelzellen, ubiquitär exprimiert wird, werden bestimmte Spleißvarianten von CD44 (CD44v) nur auf einer Untergruppe von Epithelzellen exprimiert. Die CD44-Isoformen werden durch alternatives Spleißen so erzeugt, daß die Sequenzen von 10 Exons (v1-v10) in CD44s komplett ausgeschnitten werden, jedoch bei den größeren Varianten in verschiedenen Kombinationen vorkommen können (Screaton *et al*., 1992; Heider *et al*., 1993; Hofmann *et al*., 1991). Die Varianten unterscheiden sich dadurch, daß an einer bestimmten Stelle des extrazellulären Teils des Proteins unterschiedliche Aminosäuresequenzen inseriert sind. Solche Varianten konnten in verschiedenen menschlichen Tumorzellen und in menschlichem Tumorgewebe nachgewiesen werden. So wurde kürzlich die Expression von CD44-Varianten im Verlauf der kolorektalen Karzinogenese untersucht (Heider *et al*., 1993). Die Expression von CD44-Varianten fehlt in normalem menschlichem Kolonepithel, und nur eine schwache Expression ist in den proliferierenden Zellen der Krypten nachweisbar. In späteren Stadien der Tumorprogression, z.B. in Adenokarzinomen, exprimieren alle malignen Entartungen Varianten von CD44. Weiter wurde kürzlich Expression von CD44-Spleißvarianten in aktivierten Lymphozyten sowie in Non-Hodgkin-Lymphomen gezeigt (Koopman *et al*., 1993).

Es sind verschiedene Ansätze bekannt geworden, die differentielle Expression varianter Exons des CD44-Gens in Tumoren und Normalgeweben für diagnostische und therapeutische Verfahren nutzbar zu machen (WO 94/02633, WO 94/12631, WO 95/00658, WO 95/00851, EP 0531300).

Auch die Expression varianter CD44-Moleküle in Plattenepithelkarzinomen ist schon untersucht worden. Salmi *et al*. (1993) fanden mit dem v6-spezifischen Antikörper Var3.1 eine Abnahme der v6-Expression in Tumorzellen im Vergleich zu Normalzellen. Brooks *et* *al*. (1995) erhielten mit dem v6-spezifischen Antikörper 11.9 eine heterogene Anfärbung nasopharyngealer Karzinome. Lediglich in 2/12 Fällen wurde eine starke Anfärbung erzielt, während in der Mehrzahl der Fälle nur eine schwache fokale v6-Expression immunhistologisch nachgewiesen werden konnte.

Aufgabe der vorliegenden Erfindung war die Entwicklung von neuen Verfahren zur Therapie von Plattenepithelkarzinomen sowie die Bereitstellung von Mitteln für solche Verfahren.

Diese Aufgabe konnte mit der vorliegenden Erfindung gelöst werden. Sie betrifft einen Gegenstand im Umfang der Ansprüche 1 bis 4. Sie betrifft Verfahren zur Therapie von Plattenepithelkarzinomen, die auf der Expression des varianten Exons v6 des CD44-Gens als molekularem Marker bzw. Target beruhen. Insbesondere betrifft die vorliegende Erfindung Verfahren, die auf der starken und homogenen Expression von v6 in Plattenepithelkarzinomen beruhen, die überraschend und im Gegensatz zur Lehre, wie sie aus dem Stand der Technik bekannt war, festgestellt werden konnte. Antikörpermoleküle mit entsprechender Spezifität eignen sich insbesondere als Vehikel, um Plattenepithelkarzinome *in vivo* selektiv zu erreichen.

Bevorzugt sind dabei Verfahren, die dadurch gekennzeichnet sind, daß dabei ein Antikörpermolekül verwendet wird, das die Aminosäuresequenz WFGNRWHEGYR erkennt. Besonders bevorzugt ist dabei der monoklonale Antikörper BIWA-1 (Klon VFF-18), der von einer Hybridom-Zellinie sezerniert wird, die am 7.6.1994 unter der Hinterlegungsnummer DSM ACC2174 bei der DSM-Deutsche Sammlung für Mikroorganismen und Zellkulturen GmbH, Mascheroder Weg 1b, D-38124 Braunschweig, Deutschland, hinterlegt wurde (WO 95/33771), oder Derivate dieses Antikörpers.

Weitere Aspekte der vorliegenden Erfindung sind die Verwendung solcher Antikörpermoleküle bei den erfindungsgemäßen Verfahren.

Die Nuklein- und Aminosäuresequenz des varianten Exons v6 des CD44-Gens ist bekannt (Screaton *et al*., 1992, Tölg *et al*., 1993). Die Existenz degenerierter oder alleler Varianten ist für die Ausführung der Erfindung nicht von Bedeutung; solche Varianten sind daher ausdrücklich mit eingeschlossen.

Die Sequenz von Exon v6 des menschlichen CD44-Gens ist:

Die Erfindung kann mit polyklonalen oder monoklonalen Antikörpern ausgeführt werden, die für ein Epitop spezifisch sind, das vom Exon v6 kodiert wird, insbesondere ein Epitop innerhalb der Aminosäuresequenz WFGNRWHEGYR. Die Herstellung von Antikörpern gegen bekannte Aminosäuresequenzen kann nach an sich bekannten Methoden erfolgen (Catty, 1989). Beispielsweise kann ein Peptid dieser Sequenz synthetisch hergestellt und als Antigen in einem Immunisierungsprotokoll eingesetzt werden. Ein anderer Weg ist die Herstellung eines Fusionsproteins, das die gewünschte Aminosäuresequenz enthält, indem eine Nukleinsäure (die synthetisch oder z.B. durch Polymerase-Kettenreaktion (PCR) aus einer geeigneten Probe hergestellt werden kann), die für diese Sequenz kodiert, in einen Expressionsvektor integriert und das Fusionsprotein in einem Wirtsorganismus exprimiert wird. Das gegebenenfalls gereinigte Fusionsprotein kann dann als Antigen in einem Immunisierungsprotokoll eingesetzt und Insert-spezifische Antikörper oder, im Falle monoklonaler Antikörper, Hybridome, die insertspezifische Antikörper exprimieren, mit geeigneten Verfahren selektiert werden. Solche Verfahren sind Stand der Technik. Heider *et al*. (1993, 1996a) und Koopman *et al*. (1993) beschreiben die Herstellung von Antikörpern gegen variante Epitope von CD44.

Für das erfindungsgemäße Verfahren können jedoch auch Antikörpermoleküle verwendet werden, die von poly- oder monoklonalen Antikörpern abgeleitet sind, z.B. Faboder F(ab')₂-Fragmente von Immunglobulinen, rekombinant hergestellte single-chain-Antikörper (scFv), chimäre bzw. humanisierte Antikörper sowie andere Moleküle, die spezifisch an Epitope binden, die durch Exon v6 kodiert werden. Aus dem kompletten Immunglobulin des Antikörpers BIWA-1 (VFF-18) oder anderer Antikörper können beispielsweise Faboder F(ab')₂-Fragmente oder andere Fragmente erzeugt werden (Kreitman *et al*., 1993). Der Fachmann ist ferner in der Lage, rekombinante v6-spezifische Antikörpermoleküle herzustellen. Insbesondere kann er nach Analyse der Aminosäuresequenz des Antikörpers BIWA-1 (VFF-18) und/oder unter Verwendung der Hybridom-Zellinie, die diesen Antikörper produziert, insbesondere der darin enthaltenen genetischen Information, rekombinante Antikörpermoleküle mit dem gleichen Idiotyp wie BIWA-1 (VFF-18) herstellen, d.h. Antikörpermoleküle, die im Bereich der Antigen-Bindungsstelle (*complementarity-determining regions*, CDR) die gleiche Aminosäuresequenz aufweisen wie der Antikörper BIWA-1 (VFF-18). Entsprechende Verfahren sind Stand der Technik. Solche rekombinanten Antikörpermoleküle können z.B. humanisierte Antikörper (Shin *et al*., 1989; Güssow *et* Seemann, 1991), bispezifische oder bifunktionelle Antikörper (Weiner *et al*., 1993; Goodwin, 1989, Featherstone, 1996), *single*-*chain*-Antikörper (*scFv,* Johnson *et* Bird, 1991), komplette oder fragmentarische Immunglobuline (Coloma *et al*., 1992; Nesbit *et al*., 1992; Barbas *et al*., 1992), oder durch *chain shuffling* erzeugte Antikörper (Winter *et al*., 1994) sein. Humanisierte Antikörper können beispielsweise durch CDR-grafting (EP 0239400) hergestellt werden. Auch Framework-Regionen können modifiziert werden (EP 0519596; WO 9007861). Zur Humanisierung von Antikörpern können heute Methoden wie PCR (s. z.B. EP 0368684; EP 0438310; WO 9207075) oder Computer-modelling (s. z.B. WO 9222653) angewendet werden. Es können auch Fusionsproteine, beispielsweise *single-chain*-Antikörper/Toxin-Fusionsproteine (Chaudhary *et al*., 1990; Friedman *et al*., 1993) hergestellt und verwendet werden. Unter die Oberbegriffe "Antikörper" und "Antikörpermoleküle" sollen außer polyklonalen und monoklonalen Antikörpern alle in diesem Abschnitt diskutierten Verbindungen fallen, sowie weitere Verbindungen, die sich strukturell von Immunglobulinen ableiten lassen und mit an sich bekannten Methoden herstellbar sind.

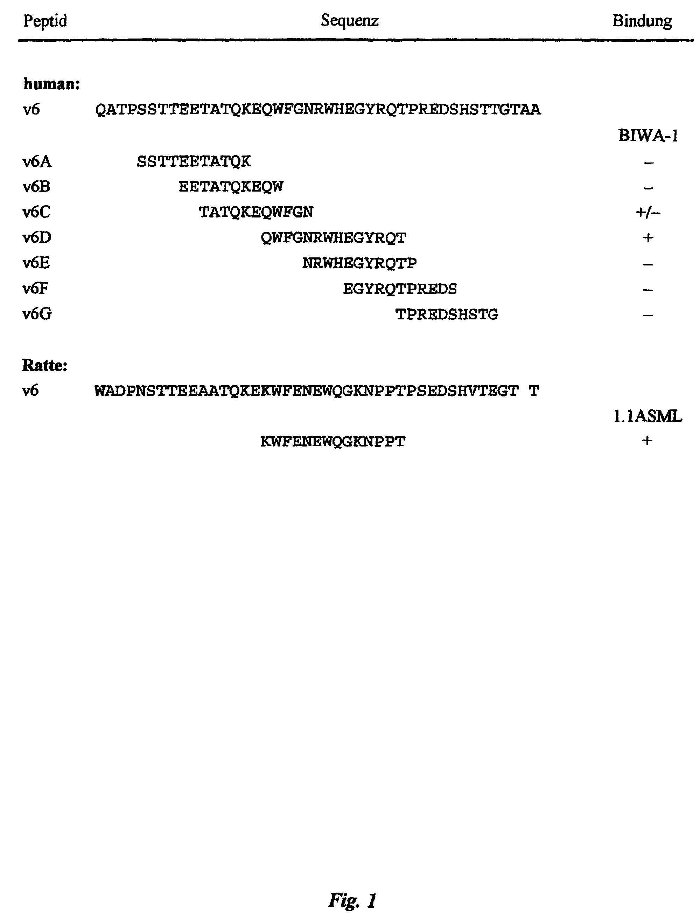

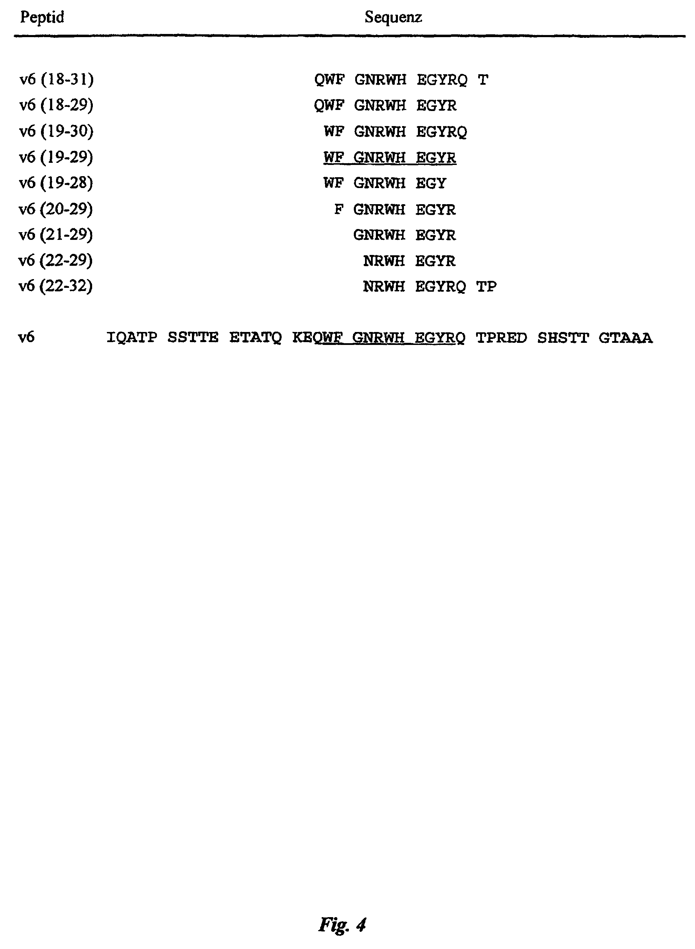

Es liegt ebenfalls im Bereich des Könnens des Durchschnittsfachmanns, in Kenntnis des Epitops (vgl. Fig. 1, Fig. 4) von BIWA-1 (VFF-18) äquivalente Antikörper mit der gleichen Bindungsspezifität herzustellen. Solche Antikörper sind daher ebenfalls in der Erfindung eingeschlossen.

Für diagnostische Verfahren können Antikörpermoleküle, vorzugsweise BIWA-1-Antikörpermoleküle, Fragmente davon oder rekombinante Antikörpermoleküle mit dem gleichen Idiotyp, beispielsweise mit radioaktiven Isotopen wie ¹²⁵I, ¹³¹I, ¹¹¹In, ^{99m}Tc oder radioaktiven Verbindungen (Larson *et al*., 1991; Thomas *et al*., 1989; Srivastava, 1988), Enzymen wie Peroxidase oder alkalischer Phosphatase (Catty *et* Raykundalia, 1989), mit Fluoreszenzfarbstoffen (Johnson, 1989) oder Biotinmolekülen (Guesdon *et al*., 1979) verknüpft werden. Für therapeutische Anwendungen können v6-spezifische Antikörpermoleküle, vorzugsweise BIWA-1(VFF-18)-Antikörpermoleküle oder VFF-18-abgeleitete Antikörpermoleküle, z.B. Fragmente davon oder rekombinante Antikörpermoleküle mit dem gleichen Idiotyp, mit Radioisotopen wie ⁹⁰Y, ¹³¹I, ¹⁸⁶Re, ¹⁸⁸Re, ¹⁵³Sm, ⁶⁷Cu, ²¹²Bi, ²¹³Bi, ¹⁷⁷Lu (Quadri *et al*., 1993; Lenhard *et al*., 1985, Vriesendorp *et al*., 1991; Wilbur *et* *al*., 1989, Maraveyas *et al*., 1995a, Jurcic *et* Scheinberg, 1994), Toxinen (Vitetta *et al*., 1991; Vitetta *et* Thorpe, 1991; Kreitman *et al*., 1993; Theuer *et al*., 1993), Zytostatika (Schrappe *et al*., 1992), Prodrugs (Wang *et al*., 1992; Senter *et al*., 1989), photoaktivierbaren Substanzen (Hemming *et al*., 1993), einem Antikörpermolekül mit einer anderen Spezifität oder radioaktiven Verbindungen verknüpft werden. Das Antikörpermolekül kann ferner mit einem Zytokin oder einem anderen immunmodulatorischen Polypeptid verknüpft sein, z.B. mit Tumornekrosefaktor, Lymphotoxin (Reisfeld *et al*., 1996) oder Interleukin-2 (Becker *et al*., 1996). Die Antikörpermoleküle können für den Einsatz in einem Pretargeting-System auch z.B. mit Streptavidin oder Biotin modifiziert werden (Goodwin, 1995).

Vorteilhaft können mit dem diagnostischen Verfahren Proben von Patienten, beispielsweise aus Biopsien, untersucht werden, bei denen der Verdacht auf Plattenepithelkarzinom besteht oder die Diagnose bereits vorliegt, der Tumor aber genauer charakterisiert werden soll. Der Nachweis varianter CD44-Moleküle, die eine Aminosäuresequenz enthalten, die vom variablen Exon v6 kodiert wird, kann auf Proteinebene mittels Antikörpern oder auf Nukleinsäureebene mittels spezifischer Nukleinsäuresonden oder Primern für die Polymerase-Kettenreaktion (PCR) erfolgen. Beispielsweise können Gewebsschnitte immunhistochemisch mit Antikörpern mit an sich bekannten Methoden untersucht werden. Aus Gewebeproben gewonnene Extrakte oder Körperflüssigkeiten können ferner mit anderen immunologischen Methoden unter Verwendung von Antikörpern untersucht werden, beispielsweise in Western Blots, Enzyme-linked Immunosorbent Assays (ELISA, Catty *et* Raykundalia, 1989), Radioimmunoassays (RIA, Catty *et* Murphy, 1989) oder verwandten Immunoassays. Die Untersuchungen können qualitativ, semiquantitativ oder quantitativ erfolgen.

Neben der *in*-*vitro*-Diagnostik eignen sich Antikörpermoleküle mit erfindungsgemäßer Spezifität auch zur in-vivo-Diagnostik von Plattenepithelkarzinomen. Trägt das Antikörpermolekül ein detektierbares Label, kann eine Detektion des Labels zu diagnostischen Zwecken, z.B. Visualisierung des Tumors *in vivo* (Imaging), oder beispielsweise zur radiounterstützten Chirurgie (*radioguided surgery*) erfolgen. Für die Verwendung von mit radioaktiven Isotopen konjugierten Antikörpern zur Immunszintigraphie (Imaging) beispielsweise gibt es eine Reihe von Protokollen, auf deren Grundlage der Fachmann die Erfindung ausführen kann (Siccardi *et al*., 1989; Keenan *et al*., 1987; Perkins *et* Pimm, 1992; Colcher *et al*., 1987; Thompson *et al*., 1984).

Durch Nachweis und/oder Quantifizierung der Expression des varianten CD44-Epitops v6 erhobene Daten können so in die Diagnose und Prognose einfließen. Vorteilhaft kann dabei die Kombination mit anderen prognostischen Parametern sein, etwa mit dem Tumorgrad.

Antikörpermoleküle mit der erfindungsgemäßen Spezifität und ggf. verknüpft mit einem zytotoxischen Agens können vorteilhaft zur Therapie von Plattenepithelkarzinomen verwendet werden. Dabei kann die Applikation systemisch oder topisch erfolgen, beispielsweise durch intravenöse (als Bolus oder Dauerinfusion), intraperitoneale, intramuskuläre, subkutane o.a. Injektion/Infusion. Protokolle für die Verabreichung von konjugierten oder nichtkonjugierten Antikörpern (sei es als komplette Immunglobuline, Fragmente, rekombinante humanisierte Moleküle o.ä.) sind Stand der Technik (Mulshine *et al*., 1991; Larson *et al*., 1991; Vitetta *et* Thorpe, 1991; Vitetta *et al*., 1991; Breitz *et al*., 1992, 1995; Press *et al*., 1989; Weiner *et al*., 1989; Chatal et *al*., 1989; Sears *et al*., 1982). Eine therapeutische Anwendung kann beispielsweise analog zu der Verwendung des Antikörpers 1.1ASML (Seiter *et al*., 1993) erfolgen. Nichtmodifizierte monoklonale Antikörper können direkt therapeutisch eingesetzt werden, wenn sie die für eine zytotoxische Wirkung geeignete intrinsische Effektorfunktion aufweisen, beispielsweise für komplement-induzierte oder antikörperinduzierte zelluläre Zytotoxizität (Riethmüller *et al*. 1994). Geeignete monoklonale Antikörper für diese Verwendung sind Maus-Antikörper vom Isotyp IgG2a oder Antikörper vom humanem IgG1-Typ. Nichtmodifizierte Antikörper können ferner zur Induktion einer patienteneigenen anti-tumoralen Reaktion über einen anti-idiotypischen Mechanismus appliziert werden (Baum *et al*., 1993; Khazaeli *et al*., 1994).

Eine bevorzugte Ausführungsform einer therapeutischen Anwendung besteht darin, ein humanisiertes v6-spezifisches Immunglobulin oder ein F(ab')₂-Fragment davon mit ⁹⁰Y (Quadri *et al*., 1993; Vriesendorp *et al*., 1995), ¹³¹I (Maraveyas *et al*., 1995a, 1995b; Juweid *et al*.*,* 1995; Press *et al*., 1995; Thomas *et al*., in: Catty 1985, S. 230-239) ¹⁸⁶Re (Breitz *et al*., 1992, 1995) oder einem anderen geeigneten Radioisotop zu verknüpfen und zur Radioimmunotherapie von Plattenepithelkarzinomen einzusetzen. Beispielsweise kann der Antikörper BIWA-1, eine humanisierte Version von BIWA-1 oder ein F(ab')₂-Fragment von BIWA-1 oder des humanisierten Antikörpers mit ⁹⁰Y unter Verwendung eines chelatbildenden Linkers wie ITCB-DTPA (Isothiocyanatbenzyl-Diethylentriaminpentaacetat) verknüpft werden, wobei eine spezifische Aktivität von 5-20 mCi/mg, vorzugsweise 10 mCi/mg erreicht werden sollte. Dieses Agens kann dann einem Patienten mit antigen-positivem Tumor in einer Dosierung von 0.1 bis 1 mCi/kg Körpergewicht, vorzugsweise 0.3 bis 0.5 mCi/kg Körpergewicht, verabreicht werden. Ist das Antikörpermolekül mit ¹³¹I verknüpft, kann bei einer spezifischen Aktivität von 2 mCi/mg ein mögliches Dosierungsschema z.B. 2 x 150 mCi im Abstand von 6 Wochen sein. Der Fachmann kann mit an sich bekannten Methoden die maximal möglichen Dosierungen ermitteln (Maraveyas *et al*., 1995a, 1995b). Bei einer zu verabreichenden Gesamtproteinmenge von 2 bis 5 mg kann die Verabreichung in Form einer schnellen intravenösen Bolusinjektion geschehen. Bei größeren Proteinmengen kann eine Infusion die günstigere Adminstrationsform sein. Bei monoklonalen Antikörpern kann es notwendig sein, das Agens vor der Verabreichung mit einem Überschuß (z.B. dem zehnfachen molaren Überschuß) des nichtradioaktiven Antikörpers zu vermischen; in diesem Fall erfolgt die Verabreichung besser in Form einer intravenösen Infusion z.B. über 15 Minuten. Die Anwendung kann wiederholt werden. Die Therapie kann mit externer Strahlentherapie kombiniert werden. Sie kann ferner durch Knochenmarkstransplantation unterstützt werden; dies ist insbesondere dann notwendig, wenn bei der Therapie eine Dosis von mehr als 1.6 Gy im Knochenmark erreicht wird.

Erfindungsgemäße Antikörpermoleküle können auch *ex vivo* zur Reinigung von CD34-positiven Stamm- und Vorläuferzellpräparationen (Immunopurging) verwendet werden. Die Strahlen- oder Chemotherapie von Plattenepithelkarzinomen kann durch autologe Knochenmarkstransplantation unterstützt werden. Die dabei applizierte Präparation von hämatopoietischen Stamm- und Vorläuferzellen muß frei von Tumorzellen sein. Dies kann durch Inkubation mit erfindungsgemäßen Antikörpermolekülen, beispielsweise Antikörper-Toxin-Konjugaten, erreicht werden (Myklebust *et al*., 1994; DE P 196 48 209.7).

Erfindungsgemäße Antikörpermoleküle können ferner in Form rekombinanter Konstrukte in den T-Zell-Rezeptor von T-Lymphozyten eingeführt werden. Solche reprogrammierten T-Lymphozyten binden selektiv an die Antigen exprimierenden Tumorzellen und entfalten zytotoxische Wirkung, sodaß sie zur Therapie von Plattenepithelkarzinomen verwendet werden können (PCT/EP9604631; Altenschmidt *et al*., 1996).

### Abbildungen

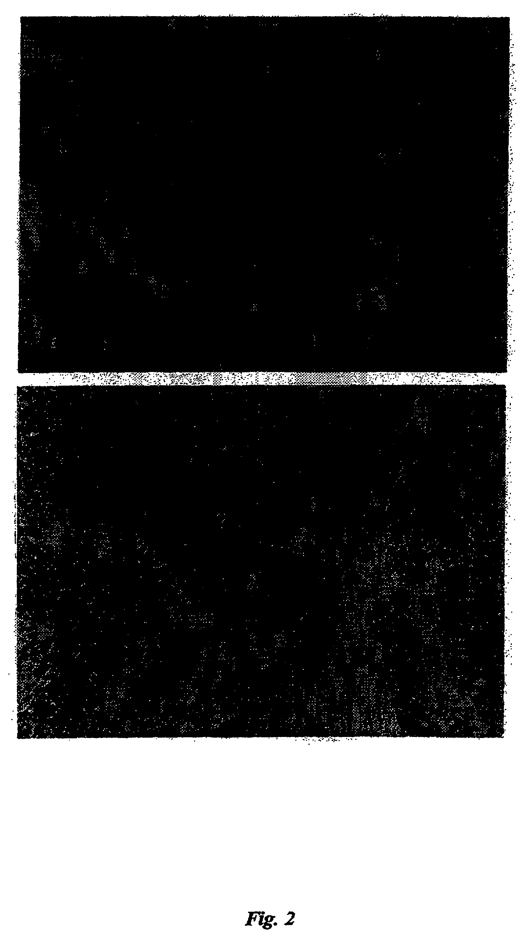

***Fig. 1:*** *Bestimmung der Epitop-Spezifität von BIWA-1 durch Bindung an synthetische Peptide, die aus der humanen CD44v6-Sequenz abgeleitet wurden.* Das korrespondierende Peptid von Ratten-CD44v6 wurde mit dem Antikörper 1.1ASML getestet. Die Bindung wurde in einem ELISA bestimmt, wobei die Peptide auf Mikrotiterplatten immobilisiert wurden (vgl. Heider *et al*., 1996b, Fig. 2). -: keine Bindung, +/-: schwache Bindung, +: starke Bindung.

***Fig. 2:*** *Immunhistochemische Analyse eines Plattenepithelkarzinoms des Larynx (a) und einer Lebermetastase eines Karzinoms des Oesophagus (b) mit dem CD44v6-spezifischen monoklonalen Antikörper BIWA-1.* In beiden Fällen kann die Reaktivität des Antikörpers mit der Membran der Tumorzellen gesehen werden. Originalvergrößerung 40x, Gegenfärbung Hämatoxylin.

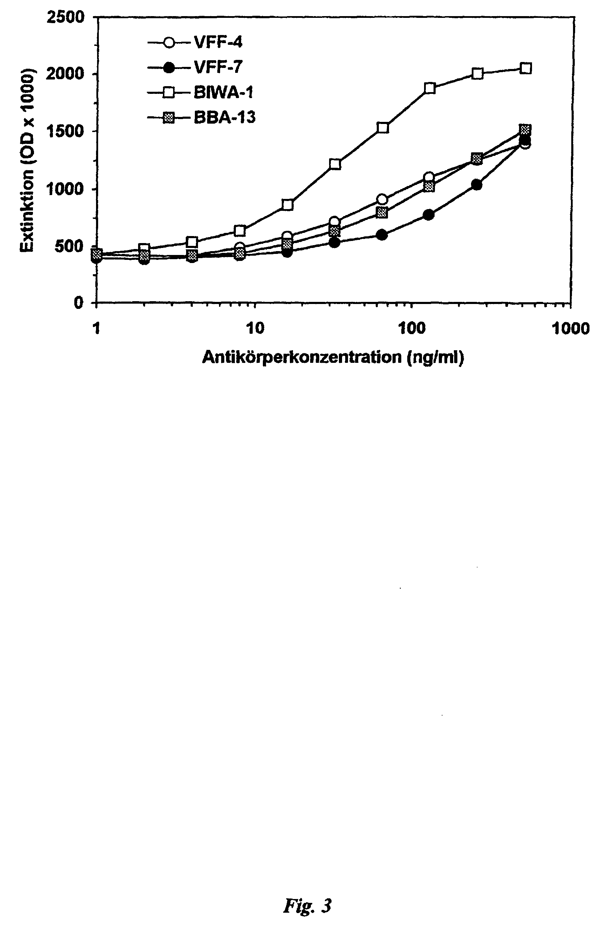

***Fig. 3:*** *Vergleich der Antigen-Bindung verschiedener CD44v6-spezifischer mAbs.* Die Bindung vier verschiedener CD44v6-spezifischer mAbs an menschliche SCC A-431-Zellen wurde in einem Zell-ELISA gemessen. MAb BIWA-1 zeigt eine höhere Affinität für die Tumorzellen als die anderen mAbs.

***Fig. 4:*** *Verfeinerte Epitop-Kartierung des mAb BIWA-1.* Die Bindung von BIWA-1 an verschiedene überlappende synthetische Peptide, die die Aminosäuren 18-32 der CD44v6-kodierten Region umspannen, wurde in einem kompetitiven ELISA gemessen. Die minimale Bindungssequenz (Peptid v6 (19-29)) ist unterstrichen.

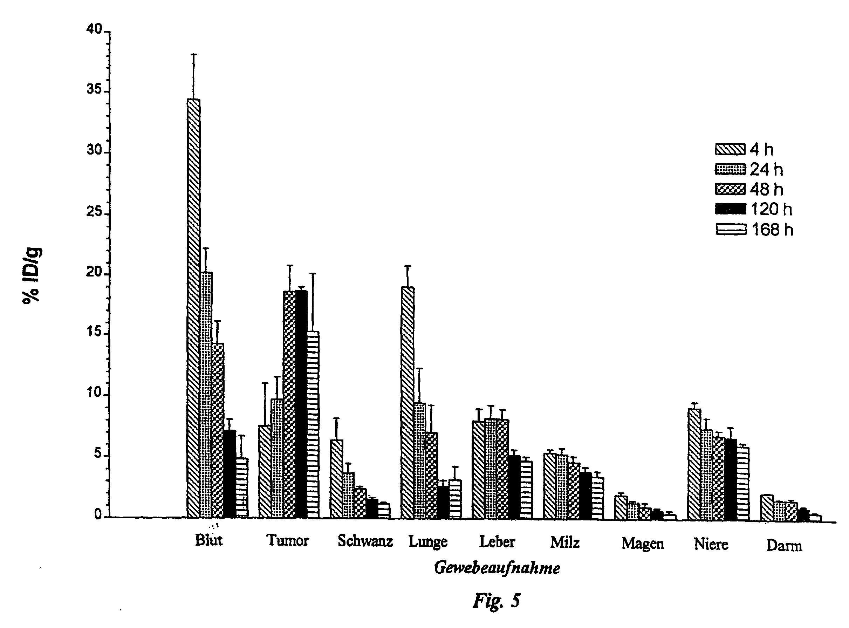

***Fig. 5:*** *Biodistribution von* ^{*125*}*I-BIWA-1 in A-431 xenotransplantierten Nacktmäusen.* Die Akkumulation des Antikörpers ist dargestellt als % ID/g (Mittelwert ± SEM) bei 4, 24, 48, 120 und 168 h post Injektion.

### Beispiele

### Beispiel 1: Expression von CD44v6 in Plattenepithelkarzinomen

### Gewebe

Insgesamt 126 Fälle Paraffin eingebetteter Tumorproben wurden immunhistochemisch mit dem mAb BIWA-1 (Klon VFF-18) auf Expression von CD44v6 analysiert. Die Proben schlossen 31 Fälle primärer Plattenepithelkarzinome (15 Fälle Larynx, 16 Fälle Haut), 91 Fälle von Lymphknotenmetastasen (Larynx, n=38; Lunge, n=27; Oesophagus, n=11; Mundhöhle, n=11; Tonsille, n=4), und 4 Fälle von Lebermetastasen (Oesophagus) ein.

### Antikörper

Die gesamte variante Region des HPKII-Typs von CD44v (Hofinann *et al*., 1991) wurde aus menschlicher Keratinozyten-cDNA durch Polymerase-Kettenreaktion (PCR) amplifiziert. Die beiden PCR-Primer *5'*-CAGGCTGGGAGCCAAATGAAGAAAATG-*3'*, Positionen 25-52, und *5*'-TGATAAGGAACGATTGACATTAGAGTTGGA-*3*', Positionen 1013-984 der LCLC97-varianten Region, wie von Hofinann *et al.* beschrieben, enthielten eine *Eco*RI-Erkennungsstelle, die benutzt wurde, um das PCR-Produkt direkt in den Vektor pGEX-2T (Smith *et al*., 1988) zu klonieren. Das resultierende Konstrukt (pGEX CD44v HPKII, v3-v10) kodiert für ein Fusionsprotein von ∼70 kD, bestehend aus Glutathion-S-transferase von *Schistosoma japonicum* und den Exons v3-v10 von humanem CD44 (Fig. 1; Heider *et al*., 1993). Das Fusionsprotein wurde in *E*. *coli* exprimiert und anschließend über Glutathion-Agarose affinitätsgereinigt (Smith *et al*., 1988).

Weibliche Balb/c Mäuse wurden intraperitoneal mit dem affinitätsgereinigten Fusionsprotein nach folgendem Schema immunisiert:
1. Immunisierung: 90 µg Fusionsprotein in komplettem Freund'schen Adjuvans
2. und 3. Immunisierung: 50 µg Fusionsprotein in inkomplettem Freund'schen Adjuvans.

Die Immunisierungen erfolgten im Abstand von jeweils 4 Wochen. 14 Tage nach der letzten Immunisierung wurden die Tiere noch an drei aufeinanderfolgenden Tagen mit jeweils 10 µg Fusionsprotein in PBS immunisiert. Am darauffolgenden Tag wurden Milzzellen eines Tieres mit hohem Antikörpertiter mit P3.X63-Ag8.653-Mausmyelomzellen mit Hilfe von Polyethylenglykol 4000 fusioniert. Die Hybridomzellen wurden dann in Mikrotiterplatten in HAT-Medium selektioniert (Köhler *et* Milstein, 1975; Kearney *et al*., 1979).

Die Bestimmung des Antikörpertiters im Serum bzw. das Screening der Hybridomüberstände wurde mit Hilfe eines ELISAs durchgeführt. Bei diesem Test wurden zunächst Mikrotiterplatten mit Fusionsprotein (GST-CD44v3-10) oder nur mit Glutathion-S-Transferase beschichtet. Anschließend wurde mit seriellen Verdünnungen von Serumproben bzw. Hybridomüberständen inkubiert und die spezifischen Antikörper mit Peroxidase-konjugierten Antikörpern gegen Maus-Immunglobulin nachgewiesen. Hybridome, die nur mit Glutathion-S-transferase reagierten, wurden verworfen. Die verbleibenden Antikörper wurden zunächst in einem ELISA mit domänenspezifischen Fusionsproteinen (Exon v3, Exon v5 + v6, Exon v6 + v7, Exon v8 - v10) charakterisiert (Koopman *et al*., 1993). Ihre immunhistochemische Reaktivität wurde an menschlichen Hautschnitten getestet.

BIWA-1 (VFF-18; Herstellung und Eigenschaften siehe auch WO 95/33771) band nur an Fusionsproteine, die eine Domäne enthielten die durch das Exon v6 kodiert wurde. Um das Epitop des Antikörpers weiter einzugrenzen, wurden verschiedene synthetische Peptide, die Teile der v6-Domäne repräsentierten, in ELISA-Bindungsassays benutzt (Fig. 1). Das 14 Aminosäure-Peptid v6D zeigte die stärkste Bindung. Folglich liegt das Epitop von BIWA-1 ganz oder teilweise innerhalb der Sequenz QWFGNRWHEGYRQT der Domäne, die von Exon v6 kodiert wird. Diese Sequenz ist homolog zum Bindungsepitop des Antikörpers 1.1ASML, der in einem therapeutischen Rattenmodell verwendet wurde, und der für Ratten-CD44v6 spezifisch ist (Fig .1).

### Immunhistochemie

Vor der Inkubation mit dem Primärantikörper wurden Paraffinschnitte (4 µm) in Rotihistol (Roth, Deutschland) 3 mal für jeweils 10 Minuten deparaffiniert und dann in einer aufsteigenden Alkoholreihe rehydriert. Die Schnitte wurden kurz mit *A. dest*. gewaschen und danach in einem Mikrowellenofen (Sharp Model R-6270) 3 mal für jeweils 10 Minuten bei 600 Watt in 0.01 M Na-Citrat-Puffer gekocht. Nach jeder Mikrowellen-Inkubation wurden die Schnitte 20 Minuten lang abgekühlt. Nach dem letzten Kühlschritt wurden die Träger in PBS gewaschen und mit normalem Ziegenserum (10% in PBS) präinkubiert. Nach 3 Waschungen in PBS wurden die Schnitte mit Primärantikörper (BIWA-1: 5 µg/ml; Maus-IgG (Isotyp-entsprechende Negativkontrolle) 5 µg/ml in PBS/1% BSA) für 1 Stunde inkubiert. Als Positivkontrolle für die Färbungsreaktion wurden normale menschliche Hautschnitte benutzt, da Keratinozyten eine CD44-Isoform exprimieren, die v3-v10 enthält. Endogene Peroxidasen wurden mit 0.3 % H₂O₂ in PBS blockiert, und die Schnitte wurden mit dem biotinylierten Sekundärantikörper (Anti-Maus IgG-F(ab')₂, DAKO Corp.) für 30 Minuten inkubiert. Zur Farbentwicklung wurden die Schnitte für 30 Minuten mit Meerrettich-Peroxidase, die an Biotin als Streptavidin-Biotin-Peroxidase-Komplex gekoppelt war (DAKO Corp.), inkubiert. Die Schnitte wurden dann in 3,3-Amino-9-ethyl-carbazol-Substrat (Sigma Immunochemicals) für 5-10 Minuten inkubiert, die Reaktion wurde mit H₂O gestoppt und die Schnitte wurden mit Hämatoxylin gegengefärbt. Die Auswertung der Färbungen wurden mit einem Zeiss-Axioskop-Lichtmikroskop durchgeführt, und die Färbungsintensitäten wurden wie folgt quantifiziert: +++, starke Expression; ++, moderate Expression; +, schwache Expression; -, uneindeutige oder keine Expression detektierbar. Nur Tumorzellen mit einer klaren Membranfärbung wurden als positiv bewertet. Der Prozentsatz positiver Tumorzellen in jedem Schnitt wurde grob abgeschätzt und zwei Gruppen wurden gebildet: fokal positive Tumoren (weniger als 10 % der Tumorzellen reagierten mit dem Antikörper) und positive Tumoren (10 oder mehr % der Tumorzellen positiv). Wenn weniger als 80% der Tumorzellen in den positiven Zellen mit dem Antikörper reagierten, wurde die entsprechende Prozentzahl angezeigt.

126 Fälle von Plattenepithelkarzinomen verschiedener Herkunft wurden mit dem CD44v6-spezifischen monoklonalen Antikörper BIWA-1 analysiert. Expression CD44v6 enthaltender Isoformen wurde in allen außer einer Tumorprobe beobachtet. Der Großteil der Proben zeigte eine Expression des Antigens auf 80-100 % der Tumorzellen, die Färbung war auf die Membran der Tumorzellen beschränkt. Mit Stromagewebe, Lymphozyten, Muskelzellen oder Endothel wurde keine Reaktion beobachtet.

Um die Expression von CD44v6-Molekülen auf diesen Tumorzellen zu quantifizieren, wurden Schnitte von normaler menschlicher Haut parallel zu den Tumorschnitten gefärbt. Normale Hautkeratinozyten exprimieren hohe Spiegel von CD44-Isoformen und zählen zu den am stärksten CD44v6 exprimierenden normalen Zellen, die bis heute beschrieben wurden. Deshalb wurde die Keratinozytenfarbung als Referenz genommen und als "stark" (+++) in unserem Bewertungssystem klassifiziert. In der Mehrzahl der untersuchten Tumorproben war die Färbung der Tumorzellen vergleichbar oder sogar stärker als die Färbung der Hautkeratinozyten, nur wenige Fälle zeigten schwache (3 Fälle von Lymphknotenmetastasen) oder moderate (2 Primärkarzinome, 10 Metastasen) Tumorfärbung. Die Färbungsreaktion war innerhalb eines gegebenen Tumorschnittes sehr homogen, wobei die meisten Tumorzellen des Schnittes die gleiche Färbungsintensität aufwiesen. Zwischen Primärtumoren und Metastasen wurden keine signifikanten Differenzen im CD44v6-Expressionsmuster beobachtet. Eine detaillierte Zusammenfassung der Ergebnisse zeigt Tabelle 1, Beispiele sind in Fig. 2 gezeigt.

**Tabelle 1:**

| *Expression von CD44v6 in Plattenepithelkarzinomen* | | | | | |
|---|---|---|---|---|---|
| **Probe** | | | **Tumortyp** | | **BIWA-1 Reaktivität** |
| 46937 | 86 | | Primär | Larynx | +++* |
| 4687 | 90 | | Primär | Larynx | +++ |
| 8372 | 90 | | Primär | Larynx | +++ |
| 17427 | 90 | | Primär | Larynx | +++ |
| 27298 | 90 | | Primär | Larynx | +++ |
| 46908 | 90 | | Primär | Larynx | +++ |
| 51334 | 90 | | Primär | Larynx | +++ |
| 51402 | 91 | | Primär | Larynx | +++ |
| 60414 | 91 | | Primär | Larynx | +++ |
| 61733 | 91 | | Primär | Larynx | +++ |
| 12280 | 92 | | Primär | Larynx | +++ |
| 23140 | 92 | | Primär | Larynx | +++ |
| 31792 | 92 | | Primär | Larynx | +++ |
| 32214 | 92 | | Primär | Larynx | +++ |
| 10209 | 95 | | Primär | Larynx | +++ |
| 2366 | 86 | | Primär | Haut | +++ |
| 2574 | 86 | | Primär | Haut | +++ |
| 9916 | 86 | | Primär | Haut | ++/+++ |
| 2696 | 87 | | Primär | Haut | +++ |
| 8906 | 87 | | Primär | Haut | +++ |
| 8191 | 88 | | Primär | Haut | +++ |
| 8354 | 88 | | Primär | Haut | ++ 50% |
| 11963 | 88 | | Primär | Haut | ++ |
| 5590 | 90 | | Primär | Haut | ++/+++ |
| 530 | 92 | | Primär | Haut | +++ |
| 2583 | 94 | | Primär | Haut | +++ |
| 11337 | 94 | | Primär | Haut | +++ |
| 10901 | 95 | | Primär | Haut | +++ |
| 11557 | 95 | | Primär | Haut | +++ |
| 11744 | 95 | | Primär | Haut | +++ |
| 11917 | 95 | | Primär | Haut | +++ |
| 4688 | 90 | I | Lymphknotenmetastase | Larynx | ++/+++ |
| 4688 | 90 | II | Lymphknotenmetastase | Larynx | - |
| 8374 | 90 | | Lymphknotenmetastase | Larynx | +++ |
| 17428 | 90 | | Lymphknotenmetastase | Larynx | +++ |
| 27300 | 90 | | Lymphknotenmetastase | Larynx | +++ |
| 36942 | 90 | | Lymphknotenmetastase | Larynx | +++ |
| 46909 | 90 | | Lymphknotenmetastase | Larynx | ++ |
| 51336 | 90 | | Lymphknotenmetastase | Larynx | +++ |
| 41108 | 91 | | Lymphknotenmetastase | Larynx | +++ |
| 51398 | 91 | | Lymphknotenmetastase | Larynx | +++ |
| 60416 | 91 | | Lymphknotenmetastase | Larynx | +++ |
| 61734 | 91 | | Lymphknotenmetastase | Larynx | +++ |
| 1318 | 92 | I | Lymphknotenmetastase | Larynx | +++ |
| 1318 | 92 | II | Lymphknotenmetastase | Larynx | +++ |
| 1318 | 92 | III | Lymphknotenmetastase | Larynx | +++ |
| 1318 | 92 | IV | Lymphknotenmetastase | Larynx | +++ |
| 2863 | 92 | I | Lymphknotenmetastase | Larynx | +++ |
| 2863 | 92 | II | Lymphknotenmetastase | Larynx | +++ |
| 5745 | 92 | I | Lymphknotenmetastase | Larynx | +++ |
| 5745 | 92 | II | Lymphknotenmetastase | Larynx | +++ |
| 8969 | 92 | I | Lymphknotenmetastase | Larynx | +++ |
| 8969 | 92 | II | Lymphknotenmetastase | Larynx | +++ |
| 8969 | 92 | III | Lymphknotenmetastase | Larynx | ++ |
| 8969 | 92 | IV | Lymphknotenmetastase | Larynx | +++ |
| 8969 | 92 | 2/I | Lymphknotenmetastase | Larynx | +++ |
| 8969 | 92 | 2/II | Lymphknotenmetastase | Larynx | +++ |
| 8969 | 92 | 2/III | Lymphknotenmetastase | Larynx | ++ |
| 8969 | 92 | 2/IV | Lymphknotenmetastase | Larynx | +/++ |
| 9366 | 92 | | Lymphknotenmetastase | Larynx | +++ |
| 9509 | 92 | | Lymphknotenmetastase | Larynx | +++ |
| 9566 | 92 | | Lymphknotenmetastase | Larynx | +++ |
| 12283 | 92 | | Lymphknotenmetastase | Larynx | +++ |
| 14046 | 92 | | Lymphknotenmetastase | Larynx | +++ |
| 31787 | 92 | | Lymphknotenmetastase | Larynx | +++ |
| 49228 | 92 | | Lymphknotenmetastase | Larynx | +++ 50% |
| 29228 | 93 | | Lymphknotenmetastase | Larynx | +++ |
| 29829 | 93 | | Lymphknotenmetastase | Larynx | ++ |
| 29804 | 95 | | Lymphknotenmetastase | Larynx | ++/+++ |
| 15293 | 91 | | Lymphknotenmetastase | Lunge | + 25% |
| 1667 | 92 | | Lymphknotenmetastase | Lunge | + 20% |
| 2757 | 92 | I | Lymphknotenmetastase | Lunge | +++ |
| 2757 | 92 | II | Lymphknotenmetastase | Lunge | +++ |
| 2757 | 92 | III | Lymphknotenmetastase | Lunge | +++ |
| 2757 | 92 | IV | Lymphknotenmetastase | Lunge | +++ |
| 4790 | 92 | | Lymphknotenmetastase | Lunge | +++ |
| 6168 | 92 | I | Lymphknotenmetastase | Lunge | ++ 50% |
| 6168 | 92 | II | Lymphknotenmetastase | Lunge | +++ |
| 6168 | 92 | III | Lymphknotenmetastase | Lunge | +++ |
| 6168 | 92 | IV | Lymphknotenmetastase | Lunge | +++ |
| 7206 | 92 | | Lymphknotenmetastase | Lunge | +++ |
| 7531 | 92 | I | Lymphknotenmetastase | Lunge | +++ |
| 7531 | 92 | II | Lymphknotenmetastase | Lunge | +++ |
| 7531 | 92 | III | Lymphknotenmetastase | Lunge | ++/+++ |
| 7531 | 92 | IV | Lymphknotenmetastase | Lunge | +++ |
| 10324 | 92 | | Lymphknotenmetastase | Lunge | +++ |
| 10519 | 92 | II | Lymphknotenmetastase | Lunge | +++ |
| 10519 | 92 | RMII | Lymphknotenmetastase | Lunge | +++ |
| 10958 | 92 | | Lymphknotenmetastase | Lunge | +++ |
| 11425 | 92 | I | Lymphknotenmetastase | Lunge | +++ |
| 11425 | 92 | II | Lymphknotenmetastase | Lunge | +++ |
| 13055 | 92 | | Lymphknotenmetastase | Lunge | ++/+++ |
| 13055 | 92 | II | Lymphknotenmetastase | Lunge | fokal +++ |
| 13055 | 92 | III | Lymphknotenmetastase | Lunge | +++ |
| 15663 | 92 | | Lymphknotenmetastase | Lunge | +++ |
| 16713 | 92 | | Lymphknotenmetastase | Lunge | +++ |
| 14980 | 91 | I | Lymphknotenmetastase | Oesophagus | +++ |
| 14980 | 91 | II | Lymphknotenmetastase | Oesophagus | +++ |
| 16641 | 91 | I | Lymphknotenmetastase | Oesophagus | +++ |
| 16641 | 91 | II | Lymphknotenmetastase | Oesophagus | +++ |
| 16641 | 91 | III | Lymphknotenmetastase | Oesophagus | +++ |
| 1059 | 92 | | Lymphknotenmetastase | Oesophagus | + |
| 1710 | 92 | I | Lymphknotenmetastase | Oesophagus | +++ |
| 1710 | 92 | II | Lymphknotenmetastase | Oesophagus | +++ |
| 1710 | 92 | III | Lymphknotenmetastase | Oesophagus | +++ |
| 11502 | 92 | I | Lymphknotenmetastase | Oesophagus | +++ |
| 11502 | 92 | II | Lymphknotenmetastase | Oesophagus | ++ |
| 202 | 92 | | Lymphknotenmetastase | Mundhöhle | ++60% |
| 6030 | 92 | | Lymphknotenmetastase | Mundhöhle | +/++/+++ 25% |
| 7335 | 92 | I | Lymphknotenmetastase | Mundhöhle | +++ |
| 7335 | 92 | II | Lymphknotenmetastase | Mundhöhle | +++ |
| 15324 | 92 | II | Lymphknotenmetastase | Mundhöhle | +++ 70% |
| 16164 | 92 | I | Lymphknotenmetastase | Mundhöhle | +++ |
| 16164 | 92 | II | Lymphknotenmetastase | Mundhöhle | +++ 50% |
| 16412 | 92 | | Lymphknotenmetastase | Mundhöhle | ++/+++ |
| 16836 | 92 | I | Lymphknotenmetastase | Mundhöhle | +++ |
| 16836 | 92 | II | Lymphknotenmetastase | Mundhöhle | +++ |
| 16836 | 92 | III | Lymphknotenmetastase | Mundhöhle | +++ |
| 6228 | 92 | I | Lymphknotenmetastase | Tonsille | +++ |
| 6228 | 92 | II | Lymphknotenmetastase | Tonsille | +++ |
| 6618 | 92 | | Lymphknotenmetastase | Tonsille | +++ |
| 11840 | 92 | | Lymphknotenmetastase | Tonsille | ++ |
| 14172 | 91 | 4 | Lebermetastase | Oesophagus | +++ |
| 14172 | 91 | 5 | Lebermetastase | Oesophagus | +++ |
| 4131 | 94 | 1 | Lebermetastase | Oesophagus | +/++ |
| 8438 | 94 | | Lebermetastase | Oesophagus | fokal ++/+++ |

80-100% der Tumorzellen reagierten positiv mit BIWA-1. In Fällen, in denen weniger Tumorzellen mit dem Antikörper reagierten, ist die entsprechende Prozentzahl angezeigt.

### Beispiel 2: Expression von CD44v6 in Nierenzellkarzinomen, Prostatakarzinomen und Lebermetastasen von Kolonkarzinomen

### Gewebe

Analysiert wurden 19 Fälle von Nierenzellkarzinomen (12 Fälle klarzellig, 5 Fälle chromophil, 1 Fall chromophob, 1 Onkozytom), 16 primäre Adenokarzinome der Prostata und 19 Fälle von Lymphknotenmetastasen von Prostatakarzinomen, sowie 30 Fälle von Lebermetastasen von Kolonkarzinomen.

### Antikörper

BIWA-1 (siehe Beispiel 1).

### Immunhistochemie

Ausführung siehe Beispiel 1.

Im Gegensatz zu den Plattenepithelkarzinomen konnte in der Mehrzahl der untersuchten Nierenzell- und Prostatakarzinome keine oder nur eine fokale Expression von CD44v6-Isoformen nachgewiesen werden. Im Falle einer mehr als fokalen Expression bei den Prostatakarzinomen war die Färbung vorwiegend diffus zytoplasmatisch und schwach bzw. heterogen im Vergleich zur Färbung von normalem Prostataepithel. In 50% der untersuchten Lebermetastasen von Kolonkarzinomen wurde eine mehr als fokale Expression von CD44v6 Isoformen nachgewiesen. Die Färbung in der Mehrheit der Fälle war schwach bis mittelmäßig, wobei meist weniger als 100% der Tumorzellen einer Probe eine Anfärbung mit BIWA-1 zeigte. Eine Zusammenfassung der Ergebnisse ist in Tabelle 2 wiedergegeben.

**Tabelle 2:**

| *Expression CD44v6 in Prostata-Adenokarzinomen, Nierenzellkarzinomen, und Lebermetastasen von kolorektalen Karzinomen* | | | | | |
|---|---|---|---|---|---|
| Tumortyp | | n | BIWA-1 Reaktivität | | |
| | | | negativ | fokal pos. | positiv |
| Prostata-Adenokarzinom | Primär | 16 | 8 | 3 | 5 |
| Prostata-Adenokarzinom | Lymphknotenmetastasen | 19 | 15 | 2 | 2 |
| Nierenzellkarzinom | Primär | 19 | 17 | 0 | 2 |
| Kolorektales Karzinom | Lebermetastasen | 30 | 7 | 8 | 15 |

### Beispiel 3: Charakterisierung von CD44v6-spezifischen Antikörpern

### Zellinie

Die menschliche SCC-Zellinie A-431 (spontanes epidermoides Karzinom der Vulva) wurde von der American Type Culture Collection (Rockwell MD) bezogen und gemäß der Herstellerangaben gezüchtet. Die Oberflächenexpression von CD44v6-enthaltenden Isoformen wurde durch FACS-Analyse bestimmt, wobei ein FITC-verknüpfter mAb BIWA-1 benutzt wurde.

### Analyse der kinetischen Konstanten

Die Bestimmung der Affinität und Kinetik der monoklonalen Antikörper-CD44v6-Wechselwirkung wurde durch Surface Plasmon Resonance (SPR) durchgeführt, wobei ein BIAcore 2000-System (Pharmacia Biocensor) benutzt wurde. Ein Glutathion-S-Transferase-CD44-Fusionsprotein, das die Region enthielt, die von den Exons v3-v10 kodiert wurde (GST/CD44 v3-v10), wurde auf einem CM5 Sensor-Chip immobilisiert, wobei die Amin-Kupplungs-Methode gemäß Herstellerangaben durchgeführt wurde. Antikörper in verschiedenen Konzentrationen (8-132 nM) in HBS (10 mM Hepes pH 7.4, 150 mM Natriumchlorid, 3.4 mM EDTA, 0.05% BIA core surfactant P20) wurde über die antigenspezifische Oberfläche bei einer Flußrate von 5 µl/min injiziert. Die Wechselwirkung wurde als Änderung des SPR-Signals aufgezeichnet. Die Dissoziation des Antikörpers wurde für 5 Minuten im Pufferfluß (HBS) beobachtet. Die Oberfläche des Chips wurde mit einem Einzelpuls von 15 µl 30 mM HCl regeneriert. Analyse der Daten und Berechnung der kinetischen Konstanten wurde mit der Pharmacia Biosensor BIA Evaluation Software, Version 2.1 durchgeführt.

Auf diese Weise wurde die Antigenaflinität von BIWA-1 mit anderen CD44v6-spezifischen mAbs (VFF4, VFF7, BBA-13 (IgG1, R&D Systems, Abingdon, U.K.)) verglichen. Kinetische und Affinitätskonstanten der verschiedenen Antikörper wurden jeweils in zwei unabhängigen Experimenten bestimmt. Tabelle 3 zeigt die Werte der Assoziationsraten (kₐ), Dissoziationsraten (k_{d}) und Dissoziationskonstanten (K_{d}) für die 4 mAbs. Alle mAbs zeigen ähnliche kₐ und k_{d}, mit Ausnahme von BBA-13, der einen 3-fach niedrigeren kₐ hat und VFF7, der eine signifikant höhere Dissoziationsrate (Faktor 5) im Vergleich zu den anderen mAbs zeigt. Dies resultiert in einer niedrigeren Bindungsaffinität für VFF7 und BBA-13 im Vergleich zu VFF4 und BIWA-1. BIWA-1 zeigt den niedrigsten K_{d} aller untersuchten Antikörper.

**Tabelle 3:**

| *Kinetische und Affinitätskonstanten verschiedener CD44v6-spezifischer mAbs* | | | |
|---|---|---|---|
| ***Antikörper*** | ***k***_{***a***} ***(M***^{***-1***}***s***^{***-1***}***)*** | ***k***_{***d***}***(s***^{***-1***}***)*** | ***K***_{***d***} ***(M)*** |
| VFF4 | 1.1 x 10⁵ | 2.6 x 10⁻⁵ | 2.4 x 10⁻¹⁰ |
| VFF7 | 1.1 x 10⁵ | 1.2 x 10⁻⁴ | 1.1 x 10⁻⁹ |
| BIWA-1 | 1.3 x 10⁵ | 2.2 x 10⁻⁵ | 1.7 x 10⁻¹⁰ |
| BBA-13 | 3.7 x 10⁴ | 2.3 x 10⁻⁵ | 6.2 x 10⁻¹⁰ |

### Analyse der Antikörper-Protein-Wechselwirkung durch ELISA.

CD44v6 exprimierende A-431-Zellen wurden in 96-Lochplatten (Falcon Microtest III, Becton Dickinson, Lincoln Park, NJ) in einer Zahl von 5 x 10⁴ pro Loch in RPMI 1640 mit 10% fötalem Kälberserum über Nacht bei 37°C kultiviert. Nach einer Waschung mit PBS/0.05% Tween 20 wurden die Zellen mit eiskaltem Ethanol für 1 Minute fixiert, wonach ein Waschschritt folgte. Die Inkubation mit den Primärantikörpern (VFF4, VFF7, BIWA-1, BBA-13, 1 ng/ml bis 600 ng/ml, jeweils in Assaypuffer: PBS/ 0.5 % BSA/ 0.05 % Tween 20) erfolgte für 1 Stunde bei Raumtemperatur und wurde von 3 Waschschritten gefolgt. Als Sekundärantikörper wurde ein Kaninchen-Antimaus-IgG-Meerettichperoxidase-konjugierter Antikörper (DAKO Corporation, Kopenhagen, Dänemark; Verdünnung 1:6000 in Assaypuffer) benutzt (1 h/RT). Nach 3 Waschschritten wurde die Farbentwicklung mit TMB-Lösung (Kirkegaard + Perry, Gaithersburg, USA) hervorgerufen. Die Extinktion wurde mit einem Hewlett-Packard ELISA-Reader gemessen.

Figur 3 zeigt, daß die relativen Affinitäten der Antikörper, wie sie durch BIAcore-Analyse bestimmt wurden, in ihrer Wechselwirkung mit den Tumorzellen reflektiert werden, wobei BIWA-1 klar die höchste Bindungsaffinität zeigt.

Die Proteindomäne, die durch das CD44-Exon v6 kodiert wird, besteht aus 45 Aminosäuren (Figur 4). Um das Epitop, daß von BIWA-1 erkannt wird, präziser zu definieren, wurde eine Serie synthetischer Peptide in ELISA-Assays eingesetzt. Vorabexperimente ergaben eine Bindung an ein zentral lokalisiertes 14-mer (Aminosäurenreste 18-31; Figur 4; vgl. auch Figur 1), aber nicht an Peptide außerhalb dieser Region. Eine zweite Serie von Peptiden wurden deshalb synthetisiert und in kompetitiven ELISAs getestet (Figur 4). Die Ergebnisse zeigen, daß das Peptid 19-29 (WFGNRWHEGYR) die Minimalstruktur repräsentiert, die für hochaffine Bindung erforderlich ist. Die Eliminierung der C-terminalen Argininreste ergab eine mehr als 100fach schwächere Bindung.

### Beispiel 4: Biodistribution radiojodinierter CD44v6-Antikörper in Xenotransplantat-tragenden Nacktmäusen

### A-431-Xenotransplantat Modell

Acht Wochen alte weibliche BALB/c nu/nu Nacktmäuse (B & K Universal, Renton, WA) wurden subkutan in die linksseitige Mittellinie 5 x 10⁶ kultivierte A-431-Zellen (humanes epidermoides Karzinom der Vulva) injiziert. Xenotransplantierte Tiere, die A-431-Tumoren trugen wurden für Biodistibutionsexperimente innerhalb von zwei Wochen verwendet (Tumorgewichte: 40-50 mg).

### Radiojodinierung von BIWA-1

Protein G-gereinigter mAb BIWA-1 (muriner IgG1) wurde an Streptavidin gekoppelt, wobei der heterobifunktionale Crosslinker Succinimidyl 4-(N-maleimido-methyl)cyclohexan-1-carboxylat verwendet wurde. Streptavidin-Lysyl-Reste wurden an reduzierte Antikörper-Cysteinyl-Reste geknüpft, die durch Dithiothreitol-Vorbehandlung des Antikörpers erzeugt wurden. Erhaltene 1:1-Korjungate (> 90%) wurden mit Ionenaustauschchromatographie weiter gereinigt. Für Biodistributionsexperimente wurde BIWA-1/SA über primäre Amine von Lysin mit ¹²⁵I markiert, wobei p-Jodophenyl-Markierungsreagenz (PIP; NEN Dupont, Wilmington, DE) verwendet wurde, gefolgt von dem Verfahren von Willbur et al. (1989). Markierung von BIWA-1 mit SA oder ¹²⁵I änderte nicht die Immunreaktivität oder die Pharmakokinetik des Antikörpers in Mäusen.

### Biodistributionsexperimente

Nacktmäuse, die mit menschlichen A-431-Tumoren xenotransplantiert waren, wurden 5-7 µCi ¹²⁵I auf 50 µg mAb BIWA-1 (spezifische Aktivität 0.1-0.14 mCi/mg) intravenös (i.v.) über die laterale Schwanzvene injiziert. Zeitverlaufs-Biodistributionsstudien wurden in Gruppen von n=3 Tieren pro Zeitpunkt bei 4, 24, 48, 120 und 168 h post-Injektion ausgeführt. An ausgewählten Zeitpunkten wurden Mäuse gewogen, über den retro-orbitalen Plexus ausgeblutet und durch Zervixdislokation getötet. Neun Organe und Gewebe wurden gesammelt und gewogen, Blut, Schwanz, Lunge, Leber, Milz, Magen, Nieren, Darm und Tumor. Die Radioaktivität in Geweben wurde im Vergleich mit Standards der injizierten Antikörperpräparation in einem Gammaszintillationszähler (Packard Instrument Company, Meriden, CT) gezählt, wobei das Energiefenster auf 25-80 keV für ¹²⁵I gesetzt wurde. Die Prozent injizierte Dosis/g des Gewebes wurde berechnet (% ID/g).

Vorexperimente hatten ergeben, daß BIWA-1 nicht mit murinem CD44v6-Antigen kreuzreagierte. Tabelle 4 und Figur 5 zeigen die Aufnahme von Radioaktivität in Tumoren und Normalgewebe. Iodinierter BIWA-1 zeigte eine schnelle Tumoraufhahme (7.6 % injizierte Dosis/g bei 4 h post-Injektion), die auf mehr auf 18 % ID/g bei 48 h zunahm und danach für bis zu 120 h konstant blieb. Sieben Tage post-Injektion (168 h) enthielt der Tumor immer noch 15.3 % ID/g Gewebe. Tumor:Gewebe-Verhältnisse wurden für individuelle Zeitpunkte berechnet und werden in Tabelle 4 gezeigt. Bei 24 h post-Injektion war das Tumor:Blut Verhältnis 0.48 und nahm auf 3.16 am Tag 7 zu. Aufnahme im Normalgewebe war niedrig und am wahrscheinlichsten verursacht durch Blut-Pool-Hintergrund in den Gewebebiopsien. Selektives *in*-*vivo*-Targeting von humanen SCC-Xenotransplantaten in Nacktmäusen mit ¹²⁵I-markiertem BIWA-1 zeigen, daß dieser monoklonale Antikörper ein hohes Potential als Targetingvehikel für die diagnostische und therapeutische Anwendung in SCC-Patienten aufweist.

**Tabelle 4:**

| *Tumor:Gewebe-Verhältnisse von* ^{*125*}*I-BIWA-1 in A-431-tumortragenden Nacktmäusen zu verschiedenen Zeitpunkten post Injektion* | | | | | |
|---|---|---|---|---|---|
| ***Verhältnis von Tumor zu*** | ***4 h*** | ***24 h*** | ***48 h*** | ***120 h*** | ***168 h*** |
| Blut | 0.22^{a} | 0.48 | 1.31 | 2.60 | 3.16 |
| Schwanz | 1.18 | 2.62 | 7.70 | 12.28 | 13.06 |
| Lunge | 0.40 | 1.03 | 2.65 | 7.04 | 4.82 |
| Leber | 0.94 | 1.18 | 2.28 | 3.57 | 3.24 |
| Milz | 1.40 | 1.84 | 4.00 | 4.86 | 4.42 |
| Magen | 3.89 | 7.37 | 19.40 | 25.56 | 33.96 |
| Niere | 0.82 | 1.31 | 2.72 | 2.79 | 2.53 |
| Darm | 3.54 | 6.24 | 11.94 | 19.24 | 27.78 |

| | | | | | |
|---|---|---|---|---|---|
| ^{a} Mittelwerte (n=3); SD sind < 7% | | | | | |

### Beispiel 5: Unterschiedliche Expression von CD44v6 in einer großen Zahl menschlicher Tumoren

In einer erweiterten Untersuchung wurden insgesamt 544 Tumorproben immunhistochemisch mit dem monoklonalen Antikörper BIWA 1 (Klon VFF-18) auf Expression von CD44v6 untersucht. Die Proben waren entweder Paraffin-eingebettet oder sofort nach der chirurgischen Entnahme in flüssigem Stickstoff eingefroren und bis zum Gebrauch bei -70°C aufbewahrt worden. Die folgenden Tumoren wurden analysiert: Basaliome (n=16), Adenokarzinome (AC) der Brust (n=55), AC des Kolons (n=83), Plattenepithelkarzinome (SCC) des Kopfes und Halses (n=125), Lungenkarzinome (n=120), Prostata AC (n=34), Nierenzellkarzinome (n=27), SCC der Haut (n=15) und AC des Magens (n=69). Die Gewebe wurden durch Routinechirurgie oder -biopsie erhalten, Normalgewebe wurden begleitend zu den Tumorproben erhalten. Die immunhistochemische Untersuchung wurde wie in Beispiel 1 ausgeführt.

Tabelle 5 zeigt eine Übersicht über die immunhistochemische Analyse von 397 verschiedenen Tumorproben mit dem mAb BIWA 1.

**Tabelle 5:**

| ***Expression von CD44v6 in menschlichen Tumoren*** | | | | |
|---|---|---|---|---|
| **Typ** | | **Gesamt n** | **Positive Fälle*** | |
| | | | n | % |
| Basalioma | Primärtumor | 16 | 10 | 62 |
| Brust AC | Primärtumor | 17 | 15 | 88 |
| | Lymphknoten-Metastasen | 34 | 31 | 91 |
| | Leber-Metastasen | 4 | 4 | 100 |
| Kolon AC | Lymphknoten-Metastasen | 51 | 21 | 41 |
| | Leber-Metastasen | 26 | 13 | 50 |
| | Hirn-Metastasen | 6 | 6 | 100 |
| Larynx SCC | Lymphknoten-Metastasen | 18 | 18 | 100 |
| Lunge AC | Primärtumor | 35 | 15 | 43 |
| Lunge SCC | Primärtumor | 9 | 9 | 100 |
| Ösophagus SCC | Primärtumor | 20 | 20 | 100 |
| Prostata AC | Primärtumor | 16 | 5 | 31 |
| | Lymphknoten-Metastasen | 18 | 0 | 0 |
| RCC | Primärtumor | 27 | 5 | 18 |
| SCLC | Primärtumor | 31 | 7 | 23 |
| Magen AC | Primärtumor | 22 | 15 | 68 |
| | Lymphknoten-Metastasen | 43 | 16 | 37 |
| | Leber-Metastasen | 4 | 4 | 100 |
| | **Gesamt n** | 397 | | |

| | | | | |
|---|---|---|---|---|
| *: ≥ 10 % der Tumorzellen positiv AC: Adenokarzinom; RCC: Nierenzellkarzinom SCLC: kleinzelliger Lungenkrebs; SCC: Plattenepithelkarzinom | | | | |

Bei kleinzelligen Lungenkarzinomen, Nierenzellkarzinomen und AC der Prostata wurde keine oder nur geringe Reaktivität beobachtet. Alle anderen untersuchten Tumortypen exprimierten CD44v6-enthaltende Isoformen in unterschiedlichem Ausmaß. Die meisten der untersuchten AC der Brust zeigten Reaktivität mit BIWA 1, und die getesteten SCC (Larynx, Lunge und Ösophagus) exprimierten CD44v6 in 100% aller Fälle.

Es wurden insgesamt 185 Fälle von SCC verschiedener Typen und Klassifikation auf Reaktivität mit BIWA 1 untersucht. Darin waren 67 Fälle primärer SCC (Larynx, n=15; Mundhöhle, n=16; Oropharynx, n=3; Haut, n=15), 77 Proben von Lymphknotenmetastasen (Larynx, n=12; Lunge, n=27; Ösophagus, n=11; Mundhöhle, n=6; Oropharynx, n=7; Hypopharynx, n=10; Tonsille, n=4), und 3 Proben aus Lebermetastasen (Ösophagus). Eine Übersicht über die immunhistochemische Analyse aller untersuchten SCC-Proben zeigt Tabelle 6.

**Tabelle 6:**

| ***Expression von CD44v6 in Plattenepithelkarzinomen*** | | | | | | | | |
|---|---|---|---|---|---|---|---|---|
| **Typ** | | **Gesamt n** | **Negativ** | | **Fokal pos.** | | **Positiv** | |
| | | | n | % | n | % | n | % |
| Hypopharynx | LNM | 10 | 0 | 0 | 0 | 0 | 10 | 100 |
| Oropharynx | PT | 3 | 0 | 0 | 0 | 0 | 3 | 100 |
| | LNM | 7 | 0 | 0 | 0 | 0 | 7 | 100 |
| Larynx | PT | 15 | 0 | 0 | 0 | 0 | 15 | 100 |
| | LNM | 30 | 1 | 3 | 0 | 0 | 29 | 97 |
| Lunge | PT | 18 | 2 | 11 | 0 | 0 | 16 | 89 |
| | LNM | 27 | 0 | 0 | 1 | 4 | 26 | 96 |
| Ösophagus | PT | 20 | 0 | 0 | 1 | 5 | 19 | 95 |
| | LNM | 11 | 0 | 0 | 0 | 0 | 11 | 100 |
| | LM | 3 | 0 | 0 | 0 | 0 | 3 | 100 |
| Mundhöhle | PT | 16 | 0 | 0 | 0 | 0 | 16 | 100 |
| | LM | 6 | 0 | 0 | 0 | 0 | 6 | 100 |
| Haut | PT | 15 | 0 | 0 | 0 | 0 | 15 | 100 |
| Tonsille | LNM | 4 | 0 | 0 | 0 | 0 | 4 | 100 |
| Gesamt n | | 185 | | | | | | |
| Fokal pos.: < 10 % der Tumorzellen positiv; LNM: Lymphknotenmetastasen; PT: Primärtumor; LM: Lebermetastasen | | | | | | | | |

Expression von CD44v6 enthaltenden Isoformen wurde in allen mit Ausnahme von drei Tumorproben gefunden (ein Fall Larynx, 2 Fälle Lunge). Die Mehrzahl der Proben zeigte eine Expression des Antigens auf 80 bis 100 % der Tumorzellen innerhalb eines einzigen Schnitts, wobei die Färbung hauptsächlich auf die Membran der Tumorzellen konzentriert war. Das am stärksten homogene Färbungsmuster wurde in Karzinomen des Larynx, Ösophagus und Hypopharynx gefunden, wobei die meisten Tumorzellen des Schnitts die gleiche Färbungsintensität zeigten.

### Literatur

Altenschmidt U, Kahl R, Moritz D, Schnierle BS, Gerstmayer B, Wels W, Groner B. Cytoplysis of tumor cells expressing the neu/erbB-2, erbB-3, and erbB-4 receptors by genetically targeted naive T lymphocytes. *Clinical Cancer Res. 2:* 1001-1008 (1996).
Barbas C F, Björling E, Chiodi F, Dunlop N, Cababa D, Jones T M, Zebedee S L, Persson M A A, Nara P L, Norrby E, Burton D R. Recombinant human Fab fragments neutralize human type 1 immunodeficiency virus *in vitro. Proc. Natl. Acad. Sci*. *U.S.A. 89:* 9339-9343 (1992).
Baum RP, Noujaim AA, Nanci A, Moebus V, Hertel A, Niesen A, Donnerstag B, Sykes T, Boniface G, Hör G. Clinical course of ovarian cancer patients under repeated stimulation of HAMA using MAb OC125 and B43.13. *Hybridoma 12(5):* 583-589 (1993).
Becker JC, Varki N, Gillies SD, Furukawa K, Reisfeld RA. An antibody-interleukin 2 fusion protein overcomes tumor heterogeneity by induction of a cellular immune response. *Proc. natl. Acad. Sci. USA 93:* 7826-7831 (1996).
Breitz H B, Weiden P L, Vanderheyden J-L, Appelbaum J W, Bjorn M J, Fer M F, Wolf S B, Ratcliff B A, Seiler C A, Foisie D C, Fisher D R, Schroff R W, Fritzberg A R, Abrams P G. Clinical experience with rhenium-186-labeled monoclonal antibodies for radioimmunotherapy: results of phase I trials. *J*. *Nucl. Med 33:* 1099-1112 (1992).
Breitz H B, Durham J S, Fisher D R, Weiden P L, DeNardo G L, Goodgold H M, Nelp W B. Phrmacokinetics and normal organ dosimetry following intraperitoneal rhenium-186-labeled monoclonal antibody. *J. Nucl. Med 36:* 754 (1995).
Brooks, L., Niedobitek, G., Agathanggelou, A., Farrell, P.J. The expression of variant CD44 in nasopharyngeal carcinoma is unrelated to expression of LMP-1. *Am. J. Pathol. 146(5):* 1102-12 (1995).
Catty, D (Hrsg). *Antibodies Vols. I und II.* IRL Press Oxford (1989).
Catty, D., Raykundalia, C. ELISA and related immunoassays. In: Catty, D (Hrsg). *Antibodies Vol. II.* IRL Press Oxford (1989), 97-152, s. S. 105-109.
Catty, D., Murphy, G. Immunoassays using radiolabels. In: Catty, D (Hrsg). *Antibodies Vol. II.* IRL Press Oxford (1989), 77-96.
Chatal J-F, Saccavini J-C, Gestin J-F, Thédrez P, Curtet C, Kremer M, Guerreau D, Nolibé D, Fumoleau P, Guillard Y. Biodistribution of indium-111-labeled OC 125 monoclonal antibody intraperitoneally injected into patients operated on for ovarian carcinomas. *Cancer Res. 49*: 3087-3094 (1989).
Chaudhary V K, Batra J K, Galdo M G, Willingham M C, Fitzgerald D J, Pastan I. A rapid method of cloning functional variable-region antibody genes in *Escherichia coli* as single-chain immunotoxins. *Proc. Natl. Acad*. *Sci. U.S.A. 87:* 1066 (1990).
Colcher D, Esteban J, Carrasquillo J A, Sugarbaker P, Reynolds J C, Bryant G, Larson S M, Schlom J. Complementation of intracavitary and intravenous administration of a monoclonal antibody (B72.3) in patients with carcinoma. *Cancer Res. 47:* 4218-4224 (1987).
Coloma M J, Hastings A, Wims L A, Morrison S L. Novel vectors for the expression of antibody molecules using variable regions generated by polymerase chain reaction. *J. Immunol. Methods 152:* 89-104 (1992).
Featherstone C. Bispecific antibodies: the new magic bullets. *Lancet 348:* 536 (1996).
Friedmann P N, McAndrew S J, Gawlak S L, Chace D, Trail P A, Brown J P, Siegall C B. BR96 sFv-PE40, a potent single-chain immunotoxin that selectively kills carcinoma cells. *Cancer Res. 53:* 334-339 (1993).
Gerretsen M, Visser GWM, Brakenhoff RH, van Walsum M, Snow GB, van Dongen GAMS. Complete ablation of small squamous cell carcinoma xenografts with ¹⁸⁶Re-labeled monoclonal antibody E48. *Cell Biophysics 24*/*25*: 135-141(1994).
Goodwin D A. A new appoach to the problem of targeting specific monoclonal antibodies to human tumors using anti-hapten chimeric antibodies. *J. Nucl. Med*. *Biol. 16:* 645 (1989).
Goodwin DA. Tumor pretargeting: almost the bottom line. *J. Nucl. Med. 36(5):* 876-879 (1995).
Günthert, U., Hofmann, M., Rudy, W., Reber, S., Zöller, M., Haußmann, I., Matzku, S., Wenzel, A., Ponta, H., and Herrlich, P. A new variant of glycoprotein CD44 confers metastatic potential to rat carcinoma cells. *Cell 65:* 13-24 (1991).
Guesdon, J. I., Ternynck, T., Avrameas, S. *J. Histochem. Cytochem. 27:* 1131 (1979).
Güssow D, Seemann G. Humanization of monoclonal antibodies. *Methods Enzymol. 203:* 99-121 (1991):
Heider, K.-H., Hofmann, M., Horst, E., van den Berg, F., Ponta, H., Herrlich, P., and Pals, S.T.A human homologue of the rat metastasis-associated variant of CD44 is expressed in colorectal carcinomas and adenomatous polyps. *J. Cell Biol. 120:* 227-233 (1993).
Heider, K.-H., Mulder, J.-W. R., Ostermann, E., Susani, S., Patzelt, E., Pals, S.T., Adolf, G.R. Splice variants of the cells surface glycoprotein CD44 associated with metastatic tumor cells are expressed in normal tissues of humans and cynomolgus monkeys. *Eur. J*. *Cancer 31A*:2385-2391 (1995).
Heider K H, Ratschek M, Zatloukal K, Adolf G R. Expression of CD44 isoforms in human renal cell carcinomas. *Virchows Arch. 428:* 267-273 (1996a).
Heider KH, Sproll M, Susani S, Patzelt E, Beaumier P, Ostermann E, Ahorn H, Adolf GR. Characterization of a high-affinity monoclonal antibody specific for CD44v6 as candidate for immunotherapy of squamous cell carcinomas. *Cancer Immunol. Immunother.:* in press. 1996.
Hemming AW, Davis NL, Finley RJ. Photodynamic therapy of squamous cell carcinoma: an evaluation of an anti-EGFR monoclonal antibody-prophyrin conjugate. *Society of Surgical Oncology, 46th Annual Cancer Symposium.* March 18-21, 1993, Los Angeles, CA, p. 67
Hofmann, M., Rudy, W., Zöller, M., Tölg, C., Ponta, H., Herrlich P., and Günthert, U. CD44 splice variants confer metastatic behavior in rats: homologous sequences are expressed in human tumor cell lines. *Cancer Res. 51:* 5292-5297 (1991).
Johnson, G. D. Immunofluorescence. In: Catty, D (Hrsg). *Antibodies Vol. II.* IRL Press Oxford (1989), 179-200, s. S. 180-189.
Johnson S, Bird R E. Construction of single-chain derivatives of monodonal antibodies and their production in *Escherichia coli. Methods Enzymol. 203:* 88-98 (1991).
Jurcic JG, Scheinberg DA. Recent Developments in the radioimmunotherapy of cancer. *Current Opinion in Immunology 6:* 715-721 (1994).
Juweid M, Sharkey R M, Markowitz A, Behr T, Swayne L C, Dunn R, Hansen H J, Shevitz J, Leung S-O, Rubin A D, Herskovic T, Hanley D, Goldenberg D M. Treatment of Non-Hodgkins's lymphoma with radiolabeled murine, chimeric, or humanized LL2, an anti-CD22 monoclonal antibody. *Cancer Res. (Suppl.) 55:* 5899s-5907s (1995).
Kearney, J.F., Radbruch A., Liesegang B., Rajewski K. A new mouse myeloma cell line that has lost imunoglobulin expression but permits construction of antibody-secreting hybrid cell lines. *J. Immunol. 123:* 1548 (1979).
Keenan A M, Weinstein J N, Carrasquillo J A, Bunn P A, Reynolds J C, Foon K A *et al.* Immunolymphoscintigraphy and the dose dependence of ¹¹¹In-labeled T101 monoclonal antibody in patients with cutaneous T-cell lymphoma. *Cancer Res. 47:* 6093-6099 (1987).
Khazaeli MB, Conry RM, LoBuglio AF. Human immune response to monoclonal antibodies. *J*. *Immunother. 15:* 42-52 (1994).
Köhler, G., Milstein, C. Continous culture of fused cells secreting antibody of predefined specifity. *Nature 265:* 495 (1975)
Koopman, G., Heider, K.-H., Horts, E., Adolf, G. R., van den Berg, F., Ponta, H., Herrlich, P., Pals, S. T. Activated human lymphocytes and aggressive Non-Hodgkin's lymphomas express a homologue of the rat metastasis-associated variant of CD44. *J. Exp*. *Med. 177:* 897-904 (1993).
Kreitman R J Hansen H J, Jones A L, FitzGerald D J P, Goldenberg D M, Pastan I. *Pseudomonas* exotoxin-based immunotoxins containing the antibody LL2 or LL2-Fab' induce regression of subcutaneous human B-cell lymphoma in mice. *Cancer Res. 53:* 819-825 (1993).
Larson S M, Cheung N-K V, Leibel S A. Radioisotope Conjugates. *In:* DeVita V T, Hellman S, Rosenberg S A (Hrsg.). Biologic therapy of caricer. J. B. Lippincott Comp., Philadelphia, 496-511 (1991).
Lenhard R E, Order S E, Spunberg J J, Asbell S O, Leibel S A. Isotopic immunoglobulin. A new systemic therapy for advanced Hodgkin's Disease. *J. Clin. Oncol. 3:* 1296-1300 (1985).
Maraveyas A, Myers M, Stafford N, Rowlinson-Busza G, Stewart J S W, Epenetos A A. Radiolabeled antibody combined with external radiotherapy for the treatment of head and neck cancer: Reconstruction of a theoretical phantom of the larynx for radiation dose calculation to local tissues. *Cancer Res. 55:* 1020-1027 (1995a).
Maraveyas A, Stafford N, Rowlinson-Busza G, Stewart J S W, Epenetos A A. Pharmacokinetics, biodistribution, and dosimetry of specific and control radiolabeled monoclonal antibodies in patients with primary head and neck squamous cell carcinoma. *Cancer Res. 55:* 1060-1069 (1995b).
Mulshine J L, Magnani J L, Linnoila R I: Applications of monoclonal antibodies in the treatment of solid tumors. *In:* DeVita V T, Hellman S, Rosenberg S A (Hrsg.). Biologic therapy of cancer. J. B. Lippincott Comp., Philadelphia, 563-588 (1991).
Myklebust AT, Godal A, Juell S, Pharo A, Fodstad O. Comparison of two antibody based methods for elimination of breast cancer cells from human bone morrow. *Cancer Res. 54:*209-214 (1994).
Nesbit M, Fu Z F, McDonald-Smith J, Steplewski Z, Curtis P J. Production of a functional monoclonal antibody recognizing human colorectal carcinoma cells from a baculovirus expression system. *J. Immunol. Methods 151:* 201-208 (1992).
Perkins A C, Pimm M V. A role for gamma scintigraphy in cancer immunology and immunotherapy. *Eur. J. Nucl*. *Med*. *19:* 1054-1063 (1992).
Press O W, Eary J F, Badger C C, Martin P J, Appelbaum F R, Levy R, Miller R, Brown S, Nelp W B, Krohn K A, Fisher D, DeSantes K, Porter B, Kidd P, Thomas E D, Bernstein I D. Treatment of refractory Non-Hodgkin's lymphoma with radiolabeled MB-1 (anti-CD37) antibody. *J*. *Clin. Oncol. 7:* 1027-1038 (1989).
Press O W, Eary J F, Appelbaum F R ,Martin P J, Nelp W B Glenn S, Fisher D J, Porter B, Metthews D C Gooley T, Bernstein I D. Phase II trial of ¹³¹I-B1 (anti-CD20) antibody therapy with autologous stem cell transplantation for relapsed B cell lymphomas. *Lancet 346:* 336-340 (1995).
Quadri S M, Vriesendorp H M, Leichner P K, Williams J R. Evaluation of indium-111 and yttrium-90 labeled linker immunoconjugates in nude mice and dogs. *J*. *Nucl. Med*. *34:* 938-945 (1993).
Reisfeld RA, Gillies SD, Mendelsohn J, Varki NM, Becker JC. Involvement of B lymphocytes in the growth inhibition of human pulmonary melanoma metastases in athymic nu/nu mice by an antibody-lymphotoxin fusion protein. *Cancer res. 56:* 1707-1712(1996).
Riethmüller G, Schneider-Gädicke E, Schlimok G, Schmiegel W *et al*. Randomised trial of monoclonal antibody for adjuvant therapy of resected Dukes' C colorectal carcinoma. *Lancet 343*: 1177-1183 (1994).
Salmi, M., Grön-Virta, K., Sointu, P., Grenman, R, Kalimo, H., Jalkanen S. Regulated expression of exon v6 containing isoforms of CD44 in man: Downregulation during malignant transformation of tumors of squamocellular origin. *J*. *Cell Biol. 122:* 431-442 (1993).
Sambrook, J., Fritsch E.E., Maniatis L, *Molecular cloning.* Cold Spring Harbor Laboratory Press, Cold Spring Harbor (1989).
Schrappe M, Bumol T F, Apelgren L D, Briggs S L, Koppel G A, Markowitz D D, Mueller B M, Reisfeld R A. Long-term growth suppression of human glioma xenografts by chemoimmunoconjugates of 4-desacetylvinblastine-3-carboxyhydrazide and monodonal antibody 9.2.27. *Cancer Res*. *52:* 3838-3844 (1992).
Screaton, G.R., Bell, M.V., Jackson, D.G., Comelis, F.B., Gerth, U., and Bell, J. I. Genomic structure of DNA encoding the lymphocyte homing receptor CD44 reveals at least 12 alternatively spliced exons. *Proc. Natl. Acad*. *Sci. U.S.A. 89:* 12160-12164(1992).
Sears H F, Mattis J, Herlyn D, Häyry P, Atkinson B, Ernst C, Steplewski Z, Koprowski H. Phase-I clinical trial of monocional aritibody in treatment of gastrointestinal tumours. *Lancet 1982 (1):* 762-765 (1982).
Seiter, S., Arch, R, Reber, S., Komitowski, D., Hofmann, M., Ponta, H:, Herrlich, P., Matzku, S., Zöller, M. Prevention of tumor metastasis formation by anti-variant CD44. *J*. *Exp. Med*. *177:* 443-455 (1993).
Senter P D, Schreiber G J, Hirschberg D L, Ashe S A, Hellström K E, Hellström I. Enhancement of the *in vitro* and *in vivo* antitumor activities of phosphorylated mitomycin C and etoposide derivatives by monoclonal antibody-alkaline phosphatase conjugates. *Cancer Res. 49:* 5789-5792 (1989).
Shin S-U, Morrison S L. Production and properties of chimeric antibody molecules. *Methods Enzymol. 178:* 459-476 (1989).
Siccardi A G, Buraggi G L, Callegaro L, Colella A C, DeFilippi P G *et al.* Immunoscintigraphy of adenocarcinomas by means of radiolabeled F(ab')₂ fragments of an anti-carcinoembryonic antigen monoclonal antibody: a multicenter study. *Cancer Res*. *49:* 3095-3103 (1989).
Smith, D.B., Johnson, K.S. Single-step purification of polypetides expressed in *Escherichia coli* as fusions with glutathione S-transferase. *Gene 67:* 31 (1988).
Srivastava S C (Hrsg). Radiolabeled monoclonal antibodies for imaging and therapy. *Life Sciences Series A 152,* Plenum New York (1988).
Tölg, C., Hofmann, M., Herrlich, P., and Ponta, H. Splicing choice from ten variant exons establishes CD44 variability. *Nucleic Acids. Res.* 21: 1225-1229 (1993).
Theuer C P, Kreitman R J, FitzGerald D J, Pastan I. Immunotoxins made with a recombinant form *of pseudomonas* exotoxin A that do not require proteolysis for activity. *Cancer Res. 53:* 340-347 (1993).
Thomas, G. D., Dykes, P. W., Bradwell, A. R. Antibodies for tumour immunodetection and methods for antibody radiolabeling. In: Catty, D. (Hrsg.). *Antibodies Vol. II.* IRL Press Oxford, 223-244 (1989).
Thompson C H, Stacker S A, Salehi N, Lichtenstein M, Leyden M J, Andrews J T. Immunoscintigraphy for detection of lymph node metastases from breast cancer. *Lancet* ***1984*** *(2):* 1245-1247 (1984).
Vitetta E S, Thorpe P E. Immunotoxins. In: DeVita V T, Hellman S, Rosenberg S A (Hrsg.). Biologic therapy of cancer. J. B. Lippincott Comp., Philadelphia, 482-495 (1991)
Vitetta E S, Stone M, Amlot P, Fay J, May R, Till M, Newman J, Clark P, Collins R, Cunningham D, Ghetie V, Uhr J W, Thorpe P E. Phase I immunotoxin trial in patients with B-cell lymphoma. *Cancer Res*. *51:* 4052-4058 (1991).
Vriesendorp H M, Herbst J M, Germack M A, Klein J L, Leichner P K, Loudenslager D M, Order S E. Phase I-II studies of yttrium-labeled antiferritin treatment for end stage Hodgkin's diesease, including Radiation Therapy Oncology Group 87-01. *J*. *Clin Oncol. 9:* 918-928 (1991).
Vriesendorp H M, Morton J D, Quadri S M. Review of five consecutive studies of radiolabeled immunoglobulin therapy in Hodgkins's Disease. *Cancer Res. (Suppl.) 55:* 5888s-5892s (1995).
Wang S-M, Chern J-W, Yeh M-Y, Ng J C, Tung E, Roffler S R. Specific activation of glucuronide prodrugs by antibody-targeted enzyme conjugates for cancer therapy. *Cancer Res. 52:* 4484-4491 (1992).
Weiner L M, O'Dwyer J, Kitson J, Comis R L, Frankel A E, Bauer R J, Kopnrad M S, Groves E S. Phase I evaluation of an anti-breast carcinoma monoclonal antibody 260F9-recombinant ricin A chain immunoconjugate. *Cancer Res. 49:* 4062-4067 (1989).
Wilbur, D. S., Hadley, S. W., Hylarides, M. D., Abrams, P. G., Beaumier, P. A., Morgan, A.C., Reno, J.M., Fritzberg, A.R. Development of a stable radioiodinating agent to label monoclonal antibodies for radiotherapy of cancer. *J. Nucl. Med. 30:* 216-226 (1989).
Winter, G., Griffith, A. D., Hawkins, R. E., Hoogenboom, H. R. Making antibodies by phage display technology. *Ann. Rev. Immunol. 12*, 433-455 (1994).

## Patentansprüche

1. Verwendung eines Antikörpermoleküls, das an die Aminosäuresequenz WFGNRWHEGYR bindet, zur Herstellung einer pharmazeutischen Zusammensetzung zur Therapie von Plattenepithelkarzinomen.

2. Verwendung nach Anspruch 1, **dadurch gekennzeichnet, daß** das Antikörpermolekül der monoklonale Antikörper BIWA-1 (VFF-18), der von der Hybridomzellinie mit der Hinterlegungsnummer DSM ACC2174 gebildet wird, oder ein Derivat dieses Antikörpers ist.

3. Verwendung nach Anspruch 1 oder 2, **dadurch gekennzeichnet, daß** das Antikörpermolekül ein monoklonaler Antikörper, ein Fab- oder F(ab')₂-Fragment eines Immunglobulins, ein rekombinant hergestellter Antikörper, ein rekombinant hergestellter chimärer bzw. humanisierter Antikörper, ein bifunktioneller oder ein single-chain-Antikörper (scFv) ist.

4. Verwendung nach einem der Ansprüche 1 bis 3, **dadurch gekennzeichnet, daß** das Antikörpermolekül mit einem radioaktiven Isotop, einer photoaktivierbaren Verbindung, einer radioaktiven Verbindung, einem Enzym, einem Fluoreszenzfarbstoff, einem Biotinmolekül, einem Toxin, einem Zytostatikum, einem Prodrug, einem Antikörpermolekül mit einer anderen Spezifität, einem Zytokin oder einem anderen immunmodulatorischen Polypeptid verknüpft ist.

## Claims

1. Use of an antibody molecule which binds to the amino acid sequence WFGNRWHEGYR, for the preparation of a pharmaceutical composition for the treatment of squamous cell carcinomas.

2. Use according to claim 1, **characterised in that** the antibody molecule is the monoclonal antibody BIWA-1 (VFF-18), which is formed by the hybridoma cell line with the Accession Number DSM ACC2174, or a derivative of this antibody.

3. Use according to claim 1 or 2, **characterised in that** the antibody molecule is a monoclonal antibody, a Fab- or F(ab')₂-fragment of an immunoglobulin, an antibody prepared by recombinant methods, a chimeric or humanised antibody prepared by recombinant methods, a bifunctional or a single-chain antibody (scFv).

4. Use according to one of claims 1 to 3, **characterised in that** the antibody molecule is linked to a radioactive isotope, a photoactivatable compound, a radioactive compound, an enzyme, a fluorescent dye, a biotin molecule, a toxin, a cytostatic, a prodrug, an antibody molecule with a different specificity, a cytokine or another immunomodulatory polypeptide.

## Revendications

1. Utilisation d'une molécule d'anticorps qui se lie à la séquence d'acides aminés WFGNRWHEGYR pour la production d'une composition pharmaceutique pour la thérapie des épithéliomas.

2. Utilisation selon la revendication 1 **caractérisée en ce que** la molécule d'anticorps est l'anticorps monoclonal BIWA-1 (VFF-18), qui est formé par la lignée cellulaire d'hybridomes ayant le numéro de dépôt DSM ACC2174, ou un dérivé de cet anticorps.

3. Utilisation selon la revendication 1 ou 2 **caractérisée en ce que** la molécule d'anticorps est un anticorps monoclonal, un fragment Fab ou F(ab')₂ d'une immunoglobuline, un anticorps produit par génie génétique, un anticorps chimère ou humanisé produit par génie génétique, un anticorps bifonctionnel ou un anticorps single chain (scFv).

4. Utilisation selon l'une des revendications 1 à 3 **caractérisée en ce que** la molécule d'anticorps est liée à un isotope radioactif, un composé photo-activable, un composé radioactif, une enzyme, un colorant fluorescent, une molécule de biotine, une toxine, un cytostatique, un précurseur de médicament, une molécule d'anticorps ayant une autre spécificité, une cytokine ou un autre polypeptide immunomodulateur.


### SEQUENZPROTOKOLL

(1) ALLGEMEINE ANGABEN:
   (i) ANMELDER:
      (A) NAME: Boehringer Ingelheim International GmbH
      (B) STRASSE: Rheinstrasse
      (C) ORT: Ingelheim
      (E) LAND: Deutschland
      (F) POSTLEITZAHL: 55216
      (G) TELEFON: +49-(0)-6132-772770
      (H) TELEFAX: +49-(0)-6132-774377

      (A) NAME: Forschungszentrum Karlsruhe GmbH
      (B) STRASSE: Postfach 3640
      (C) ORT: Karlsruhe
      (E) LAND: Deutschland
      (F) POSTLEITZAHL: 76021

      (A) NAME: Heider, Karl-Heinz
      (B) STRASSE: Hervicusgasse 4/3/21
      (C) ORT: Wien
      (E) LAND: Oesterreich
      (F) POSTLEITZAHL: 1120

      (A) NAME: Adolf, Guenther
      (B) STRASSE: Stiftgasse 15-17/10
      (C) ORT: Wien
      (E) LAND: Oesterreich
      (F) POSTLEITZAHL: 1070

      (A) NAME: Ostermann, Elinborg
      (B) STRASSE: Mauerbachstr. 56/6
      (C) ORT: Wien
      (E) LAND: Oesterreich
      (F) POSTLEITZAHL: 1140

      (A) NAME: Patzelt, Erik
      (B) STRASSE: Hans-Buchmueller-Gasse 8
      (C) ORT: Purkersdorf
      (E) LAND: Oesterreich
      (F) POSTLEITZAHL: 3002

      (A) NAME: Sproll, Marlies
      (B) STRASSE: Schwenkgasse 3
      (C) ORT: Wien
      (E) LAND: Oesterreich
      (F) POSTLEITZAHL: 1120
   (ii) BEZEICHNUNG DER ERFINDUNG: Verfahren zur Diagnose und Therapie von Plattenepithelkarzinomen
   (iii) ANZAHL DER SEQUENZEN: 16
   (iv) COMPUTER-LESBARE FASSUNG:
      (A) DATENTR 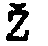 GER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
(2) ANGABEN ZU SEQ ID NO: 1:
   (i) SEQUENZKENNZEICHEN:
      (A) LÄNGE: 129 Basenpaare
      (B) ART: Nucleotid
      (C) STRANGFORM: beides
      (D) TOPOLOGIE: beides
   (ii) ART DES MOLEKÜLS: Genom-DNA
   (ix) MERKMAL:
      (A) NAME/SCHLÜSSEL: exon
      (B) LAGE:1..129
      (D) SONSTIGE ANGABEN:/product= "CD44"
         /label= v6
         /note= "GenBank data base accession No. L05411"
         /citation= ([1])
   (ix) MERKMAL:
      (A) NAME/SCHLÜSSEL: CDS
      (B) LAGE:3..128
   (x) VERÖFFENTLICHUNGSINFORMATION:
      (A) AUTORS: Screaton, GR
         Bell, MV
         Jackson, DG
         Cornelis, FB
         Gerth, U
         Bell, JI
      (B) TITEL: Genomic structure of DNA encoding the lymphocyte homing receptor CD44 reveals at least 12 alternatively spliced exons
      (C) ZEITSCHRIFT: Proc. Natl. Acad. Sci. U.S.A.
      (D) BAND: 89
      (F) SEITEN: 12160-12164
      (G) DATUM: December-1992
      (K) BELANGREICHE RESTE IN SEQ ID NO: 1: VON 1 BIS 129
   (X) VERÖFFENTLICHUNGSINFORMATION:
      (H) DOCUMENTNUMMER: DE 195 45 472.3
      (I) ANMELDETAG: 06-DEC-1995
   (x) VERÖFFENTLICHUNGSINFORMATION:
      (H) DOCUMENTNUMMER: DE 196 15 074.4
      (I) ANMELDETAG: 17-APR-1996
   (xi) SEQUENZBESCHREIBUNG: SEQ ID NO: 1:
   TC CAG GCA ACT CCT AGT AGT ACA ACG GAA GAA ACA GCT ACC CAG AAG  47
      Gln Ala Thr Pro Ser Ser Thr Thr Glu Glu Thr Ala Thr Gln Lys
       1               5                  10                  15
   GAA CAG TGG TTT GGC AAC AGA TGG CAT GAG GGA TAT CGC CAA ACA CCC 95
   Glu Gln Trp Phe Gly Asn Arg Trp His Glu Gly Tyr Arg Gln Thr Pro
                   20                  25                  30
   AGA GAA GAC TCC CAT TCG ACA ACA GGG ACA GCT G                   129
   Arg Glu Asp Ser His Ser Thr Thr Gly Thr Ala
               35                  40
(2) ANGABEN ZU SEQ ID NO: 2:
   (i) SEQUENZKENNZEICHEN:
      (A) LÄNGE: 42 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 2:
   Gln Ala Thr Pro Ser Ser Thr Thr Glu Glu Thr Ala Thr Gln Lys Glu
    1               5                  10                  15
   Gln Trp Phe Gly Asn Arg Trp His Glu Gly Tyr Arg Gln Thr Pro Arg
               20                  25                  30
   Glu Asp Ser His Ser Thr Thr Gly Thr Ala
           35                  40
(2) ANGABEN ZU SEQ ID NO: 3:
   (i) SEQUENZKENNZEICHEN:
      (A) LÄNGE: 14 Aminosäuren
      (B) ART: Aminosäure
      (C) STRANGFORM: Einzelstrang
      (D) TOPOLOGIE: linear
   (ii) ART DES MOLEKÜLS; Peptid
   (x) VERÖFFENTLICHUNGSINFORMATION:
      (H) DOCUMENTNUMMER: DE 195 45 472.3
      (I) ANMELDETAG: 06-DEC-1995
   (x) VERÖFFENTLICHUNGSINFORMATION:
      (H) DOCUMENTNUMMER: DE 196 15 074.4
      (I) ANMELDETAG: 17-APR-1996
   (xi) SEQUENZBESCHREIBUNG: SEQ ID NO: 3:
   Gln Trp Phe Gly Asn Arg Trp His Glu Gly Tyr Arg Gln Thr
   1               5                   10
(2) ANGABEN ZU SEQ ID NO: 4:
   (i) SEQUENZKENNZEICHEN:
      (A) LÄNGE: 27 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "PCR primer"
   (x) VERÖFFENTLICHUNGSINFORMATION:
      (H) DOCUMENTNUMMER: DE 195 45 472.3
      (I) ANMELDETAG: 06-DEC-1995
   (x) VERÖFFENTLICHUNGSINFORMATION:
      (H) DOCUMENTNÜMMER: DE 196 15 074.4
      (I) ANMELDETAG: 17-APR-1996
   (xi) SEQUENZBESCHREIBUNG: SEQ ID NO: 4:
   CAGGCTGGGA GCCAAATGAA GAAAATG                                    27
(2) ANGABEN ZU SEQ ID NO: 5:
   (i) SEQUENZKENNZEICHEN:
      (A) LÄNGE: 30 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "PCR primer"
   (x) VERÖFFENTLICHUNGSINFORMATION:
      (H) DOCUMENTNUMMER: DE 195 45 472.3
      (I) ANMELDETAG: 06-DEC-1995
   (x) VER™FFENTLICHUNGSINFORMATION:
      (H) DOCUMENTNUMMER: DE 196 15 074.4
      (I) ANMELDETAG: 17-APR-1996
   (xi) SEQUENZBESCHREIBUNG: SEQ ID NO: 5:
   TGATAAGGAA CGATTGACAT TAGAGTTGGA                                    30
(2) ANGABEN ZU SEQ ID NO: 6:
   (i) SEQUENZKENNZEICHEN:
      (A) LÄNGE; 11 Aminosäuren
      (B) ART: Aminosäure
      (C) STRANGFORM: Einzelstrang
      (D) TOPOLOGIE: linear
   (ii) ART DES MOLEKÜLS: Peptid
   (x) VERÖFFENTLICHUNGSINFORMATION:
      (H) DOCUMENTNUMMER: DE 195 45 472.3
      (I) ANMELDETAG: 06-DEC-1995
   (x) VERÖFFENTLICHUNGSINFORMATION:
      (H) DOCUMENTNUMMER: DE 196 15 074.4
      (I) ANMELDETAG: 17-APR-1996
   (xi) SEQUENZBESCHREIBUNG: SEQ ID NO: 6:
(2) ANGABEN ZU SEQ ID NO: 7:
   (i) SEQUENZKENNZEICHEN:
      (A) LÄNGE: 43 Aminosäuren
      (B) ART: Aminosäure
      (C) STRANGFOBM: Einzelstrang
      (D) TOPOLOGIE: linear
   (ii) ART DES MOLEKÜLS: Peptid
   (x) VERÖFFENTLICHUNGSINFORMATION:
      (H) DOCUMENTNUMMER: DE 195 45 472.3
      (I) ANMELDETAG: 06-DEC-1995
   (x) VERÖFFENTLICHUNGSINFORMATION:
      (H) DOCUMENTNÜMMER: DE 196 15 074.4
      (I) ANMELDETAG: 17-APR-1996
   (xi) SEQUENZBESCHREIBUNG: SEQ ID NO: 7:
   Gln Ala Thr Pro Ser Ser Thr Thr Glu Glu Thr Ala Thr Gln Lys Glu
   1               5                   10                  15
   Gln Trp Phe Gly Asn Arg Trp His Glu Gly Tyr Arg Gln Thr Pro Arg
               20                  25                  30
   Glu Asp Ser His Ser Thr Thr Gly Thr Ala Ala
           35                  40
(2) ANGABEN ZU SEQ ID NO: 8:
   (i) SEQUENZKENNZEICHEN:
      (A) LÄNGE: 11 Aminosäuren
      (B) ART: Aminosäure
      (C) STRANGFORM: Einzelstrang
      (D) TOPOLOGIE: linear
   (ii) ART DES MOLEKÜLS: Peptid
   (x) VERÖFFENTLICHUNGSINFORMATION:
      (H) DOCUMENTNUMMER: DE 195 45 472.3
      (I) ANMELDETAG: 06-DEC-1995
   (x) VERÖFFENTLICHUNGSINFORMATION:
      (H) DOCUMENTNUMMER: DE 196 15 074.4
      (I) ANMELDETAG: 17-APR-1996
   (xi) SEQUENZBESCHREIBUNG: SEQ ID NO: 8:
   Ser Ser Thr Thr Glu Glu Thr Ala Thr Gln Lys
   1               5                   10
(2) ANGABEN ZU SEQ ID NO: 9:
   (i) SEQUENZKENNZEICHEN:
      (A) LÄNGE: 10 Aminosäuren
      (B) ART: Aminosäure
      (C) STRANGFORM: Einzelstrang
      (D) TOPOLOGIE: linear
   (ii) ART DES MOLEKÜLS: Peptid
   (x) VERÖFFENTLICHUNGSINFORMATION:
      (H) DOCUMENTNUMMER: DE 195 45 472.3
      (I) ANMELDETAG: 06-DEC-1995
   (x) VERÖFFENTLICHUNGSINFORMATION:
      (H) DOCUMENTNUMMER: DE 196 15 074.4
      (I) ANMELDETAG: 17-APR-1996
   (xi) SEQUENZBESCHREIBUNG: SEQ ID NO: 9:
   Glu Glu Thr Ala Thr Gln Lys Glu Gln Trp
   1               5                   10
(2) ANGABEN ZU SEQ ID NO: 10:
   (i) SEQUENZKENNZEICHEN:
      (A) LÄNGE: 11 Aminosäuren
      (B) ART: Aminosäure
      (C) STRANGFORM: Einzelstrang
      (D) TOPOLOGIE: linear
   (ii) ART DES MOLEKÜLS: Peptid
   (x) VERÖFFENTLICHUNGSINFORMATION:
      (H) DOCUMENTNUMMER: DE 195 45 472.3
      (I) ANMELDETAG: 06-DEC-1995
   (x) VERÖFFENTLICHUNGSINFORMATION:
      (H) DOCUMENTNUMMER: DE 196 15 074.4
      (I) ANMELDETAG: 17-APR-1996
   (xi) SEQUENZBESCHREIBUNG: SEQ ID NO: 10:
   Thr Ala Thr Gln Lys Glu Gln Trp Phe Gly Asn
   1               5                   10
(2) ANGABEN ZU SEQ ID NO: 11:
   (i) SEQUENZKENNZEICHEN:
      (A) LÄNGE: 14 Aminosäuren
      (B) ART: Aminosäure
      (C) STRANGFORM: Einzelstrang
      (D) TOPOLOGIE: linear
   (ii) ART DES MOLEKÜLS: Peptid
   (x) VERÖFFENTLICHUNGSINFORMATION:
      (H) DOCUMENTNUMMER: DE 195 45 472.3
      (I) ANMELDETAG: 06-DEC-1995
   (x) VERÖFFENTLICHUNGSINFORMATION:
      (H) DOCUMENTNUMMER: DE 196 15 074.4
      (I) ANMELDETAG: 17-APR-1996
   (xi) SEQUENZBESCHREIBUNG: SEQ ID NO: 11:
   Gln Trp Phe Gly Asn Arg Trp His Glu Gly Tyr Arg Gln Thr
   1               5                   10
(2) ANGABEN ZU SEQ ID NO: 12:
   (i) SEQUENZKENNZEICHEN:
      (A) LÄNGE: 11 Aminosäuren
      (B) ART: Aminosäure
      (C) STRANGFORM: Einzelstrang
      (D) TOPOLOGIE: linear
   (ii) ART DES MOLEKÜLS: Peptid
   (x) VERÖFFENTLICHUNGSINFORMATION:
      (H) DOCUMENTNUMMER: DE 195 45 472.3
      (I) ANMELDETAG: 06-DEC-1995
   (x) VERÖFFENTLICHUNGSINFORMATION:
      (H) DOCUMENTNUMMER: DE 196 15 074.4
      (I) ANMELDETAG: 17-APR-1996
   (xi) SEQUENZBESCHREIBUNG: SEQ ID NO: 12:
   Asn Arg Trp His Glu Gly Tyr Arg Gln Thr Pro
   1               5                   10
(2) ANGABEN ZU SEQ ID NO: 13:
   (i) SEQUENZKENNZEICHEN:
      (A) LÄNGE: 11 Aminos,,uren
      (B) ART: Aminos,,ure
      (C) STRANGFORM: Einzelstrang
      (D) TOPOLOGIE: linear
   (ii) ART DES MOLEKÜLS: Peptid
   (x) VERÖFFENTLICHUNGSINFORMATION:
      (H) DOCUMENTNUMMER: DE 195 45 472.3
      (I) ANMELDETAG: 06-DEC-1995
   (x) VERÖFFENTLICHUNGSINFORMATION:
      (H) DOCUMENTNUMMER: DE 196 15 074.4
      (I) ANMELDETAG: 17-APR-1996
   (xi) SEQUENZBESCHREIBUNG: SEQ ID NO: 13:
   Glu Gly Tyr Arg Gln Thr Pro Arg Glu Asp Ser
   1               5                   10
(2) ANGABEN ZU SEQ ID NO: 14:
   (i) SEQUENZKENNZEICHEN:
      (A) LÄNGE: 10 Aminosäuren
      (B) ART: Aminosäure
      (C) STRANGFORM: Einzelstrang
      (D) TOPOLOGIE: linear
   (ii) ART DES MOLEKÜLS: Peptid
   (x) VERÖFFENTLICHUNGSINFORMATION:
      (H) DOCUMENTNUMMER: DE 195 45 472.3
      (I) ANMELDETAG: 06-DEC-1995
   (x) VERÖFFENTLICHUNGSINFORMATION:
      (H) DOCUMENTNUMMER: DE 196 15 074.4
      (I) ANMELDETAG: 17-APR-1996
   (xi) SEQUENZBESCHREIBUNG: SEQ ID NO: 14:
   Thr Pro Arg Glu Asp Ser His Ser Thr Gly
   1               5                   10
(2) ANGABEN ZU SEQ ID NO: 15:
   (i) SEQUENZKENNZEICHEN:
      (A) LANGE: 42 Aminosäuren
      (B) ART: Aminosäure
      (C) STRANGFORM: Einzelstrang
      (D) TOPOLOGIE: linear
   (ii) ART DES MOLEKÜLS: Peptid
   (x) VERÖFFENTLICHUNGSINFORMATION:
      (H) DOCUMENTNUMMER: DE 195 45 472.3
      (I) ANMELDETAG: 06-DEC-1995
   (x) VERÖFFENTLICHUNGSINFORMATION:
      (H) DOCUMENTNUMMER: DE 196 15 074.4
      (I) ANMELDETAG: 17-APR-1996
   (xi) SEQUENZBESCHREIBUNG: SEQ ID NO: 15:
   Trp Ala Asp Pro Asn Ser Thr Thr Glu Glu Ala Ala Thr Gln Lys Glu
   1               5                   10                  15
   Lys Trp Phe Glu Asn Glu Trp Gln Gly Lys Asn Pro Pro Thr Pro Ser
               20                  25                  30
   Glu Asp Ser His Val Thr Glu Gly Thr Thr
           35                  40
(2) ANGABEN ZU SEQ ID NO: 16:
   (i) SEQUENZKENNZEICHEN:
      (A) LÄNGE: 14 Aminosäuren
      (B) ART: Aminosäure
      (C) STRANGFORM: Einzelstrang
      (D) TOPOLOGIE: linear
   (ii) ART DES MOLEKÜLS: Peptid
   (x) VERÖFFENTLICHUNGSINFORMATION:
      (H) DOCUMENTNUMMER: DE 195 45 472.3
      (I) ANMELDETAG: 06-DEC-1995
   (x) VERÖFFENTLICHUNGSINFORMATION:
      (H) DOCUMENTNUMMER: DE 196 15 074.4
      (I) ANMELDETAG: 17-APR-1996
   (xi) SEQUENZBESCHREIBUNG: SEQ ID NO: 16:
   Lys Trp Phe Glu Asn Glu Trp Gln Gly Lys Asn Pro Pro Thr
   1               5                   10